# EUROPEAN PATENT SPECIFICATION

(11) **EP 2 289 975 B1**
(45) Date of publication and mention of the grant of the patent: **22.08.2012**
(21) Application number: 10251500.4
(22) Date of filing: 26.08.2010
(51) Int. Cl.: C08G 77/26, A61K 8/27, A61K 8/29, A61K 8/898, A61Q 5/00, A61Q 19/00, C08K 9/06

(54) **Organopolysiloxane compound and amidoamine compound, and cosmetic preparation**
Organpolysiloxanverbindung und Amidoaminverbindung sowie kosmetische Zubereitung
Composé organopolysiloxane, composé amidoamine et préparation cosmétique

(30) Priority: 27.08.2009 JP 2009196947; 27.08.2009 JP 2009197015
(43) Date of publication of application: 02.03.2011
(73) Proprietor: Shin-Etsu Chemical Co., Ltd., Tokyo (JP)
(72) Inventor: Moriya, Hiroyuki, Annaka-shi, Gunma-ken (JP); Kamei, Masanao, Annaka-shi, Gunma-ken (JP); Ikeda, Teruki, Annaka-shi, Gunma-ken (JP)
(74) Representative: Stoner, Gerard Patrick

(56) References cited:
- WO-A1-2005/111115
- US-A- 6 124 490
- US-A1- 2002 058 776
- US-A1- 2002 114 771
- US-A1- 2007 207 176

## Description

This invention relates to an organopolysiloxane compound containing a branched structure and having a pyrrolidone carboxyl group and also to an amidoamine compound obtained from the organopolysiloxane compound. The invention also relates to a cosmetic preparation formulated with the organopolysiloxane compound that has excellent emulsifying properties relative to oils such as silicone oils, hair treating properties, and powder treating properties.

### BACKGROUND

Organopolysiloxanes have been used in all the fields of industries including electric, electronic, automotive, OA equipment, medical, cosmetic, food, architectural and the like industries. Requirement characteristics and manners for use in the respective fields are rich in diversity, for which many polysiloxanes modified with a variety of organic groups at the siloxane skeleton have been developed. Organopolysiloxanes containing a carboxyl group are characteristic of their reactivity, electrolytic property and high hydrophilicity and are useful for resin modification and as a treating agent for fibers, hair, paper and powder and also as an emulsifier such as for silicone oils.

As an organopolysiloxane containing a carboxyl group, the following compounds are known:

R₃Si-R'-COOH

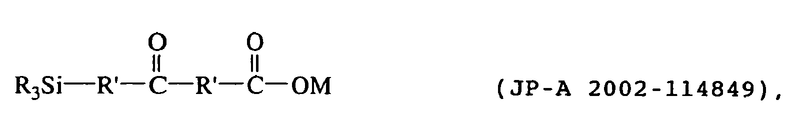

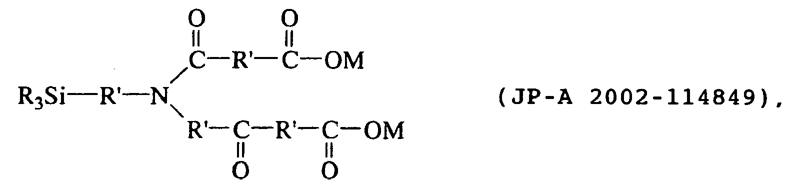

R₃Si-(RO)ₙ-R'-(OR)ₙ-COOM (JP-A 2004-307520), 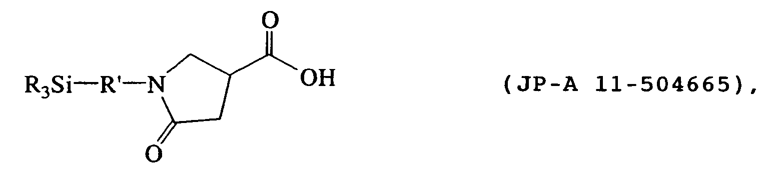 wherein R is an alkyl group and R' is an alkylene group.

The process of preparing the carboxyl group-containing organopolysiloxane is described in JP-A 11-504665 wherein a pyrrolidone carboxyl group-containing organopolysiloxane is obtained by the reaction between a primary amino group-containing organopolysiloxane and itaconic acid. This organopolysiloxane is commercially sold under the name of Monasil PCA from Uniquema Corp. For cosmetic preparations making use of this compound, it has been proposed to formulate the compound in hair cosmetic preparations (JP-A 2005-132779), to use it as a dispersant for metal oxide (JP-A 2007-513934) and to formulate it in solid powdery cosmetic preparations (JP-A 2006-169206).

As stated above, those carboxyl group-containing organopolysiloxanes differ in atomic group bonding the silicon atom and the carboxyl group, and the disclosed ones have an ester bond, an amide bond or an ether bond. Among them, one having an N-hetero ring is shown in JP-A 11 - 504665, and this organopolysiloxane has a polysiloxane structure that is linear, so that the polysiloxane serving as a hydrophobic moiety in the molecule upon surface treatment of fibers, hair, paper, inorganic fillers and the like is not three-dimensionally extended, with the possibility that the effect thereof becomes unsatisfactory. Especially, the formulation of this organopolysiloxane in cosmetic preparations has presented a problem in that it is unsatisfactory for use as a treating agent of the hair or powder and becomes greasy upon use as an emulsified cosmetic, thus not making makeup last longer.

The invention has been made under such circumstances. The present invention provides a pyrrolidonecarboxyl group-containing organopolysiloxane compound having a siloxane branched structure for avoiding such drawbacks as described above and also provides an amidoamine compound. Another aspect is a cosmetic preparation which is longer lasting and has reduced greasiness.

We have made intensive studies in order to achieve the above objects and, as a result, found that it is effective to introduce SiO₂ units (Q units) and/or R¹SiO_{3/2} units (T units) into an organopolysiloxane having a group represented by the following formula (1): 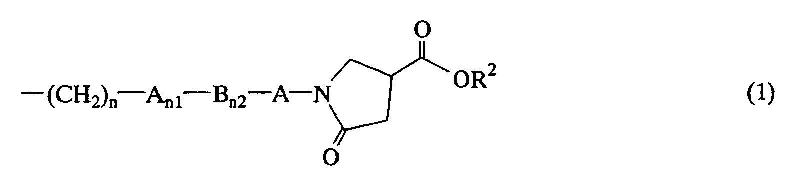 wherein R² represents a hydrogen atom, an alkyl group having 1 to 6 carbon atoms or an alkali metal, A's may be the same or different and represent a linear or branched alkylene group having 1 to 12 carbon atoms, B represents -NR³- wherein R³ represents a hydrogen atom or an alkyl group having 1 to 6 carbon atoms, or sulfur or oxygen atom, n is 0 or 2, n1 is 0 or 1, n² is 0 or 1 provided that when n is 0 and n2 is 1, n1 is 1, when n is 2 and n2 is 1, n1 is 0 or 1, and when n is 2 and n2 is 0, n1 is 0. Especially, when at least one of R¹'s of the T units is provided as the group of the above formula (1) and the resultant compound is formulated in cosmetic preparations, there can be obtained cosmetic preparations that ensure such effective treatments of powder and hair as will not be achieved by the use of linear organopolysiloxanes. Moreover, the cosmetic preparations do not become greasy and ensure good skin adherability and makeup lasting while feeling dry.

In more details, the organopolysiloxanes indicated in the above prior art patent documents are such that all the silicon atoms joined to the pyrrolidonecarboxyl group-containing organic group are in the form of a monosiloxane unit of the following formula (4) or a disiloxane unit of the following formula (5). The silicon atom joined to a trisiloxane unit of the following formula (6) has never been known up to now. In the following formulas, the bonding site extending from the oxygen atom binds to other silicon atom. The organopolysiloxane of the invention is **characterized in that** because the organopolysiloxane has a pyrrolidonecarboxyl group joined to at least one trisiloxy unit in the molecule, there is provided such a structure that polysiloxane chains extend in three directions from the silicon atom to which an organic group including the pyrrolidonecarboxyl polar group is bound. Such a bulky polysiloxane structure is established in the vicinity of the polar group, for which it has been found that when the organopolysiloxane is formulated in cosmetic preparations, there can be realized an effective powder and hair treatment as will not be expected with the use of linear organopolysiloxanes having the structures of the formulas (4) and (5), along with the formation of emulsions having high emulsification stability. In addition, there can also be provided cosmetic preparations that are not greasy and ensure good skin adherability and makeup lasting performance while feeling dry. 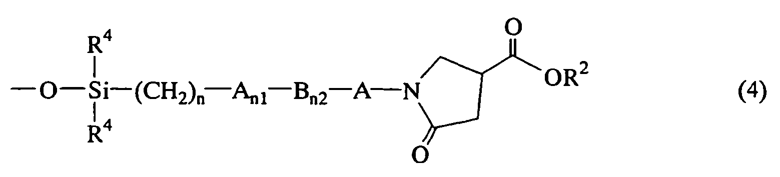 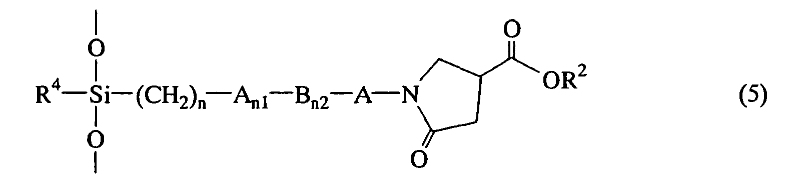 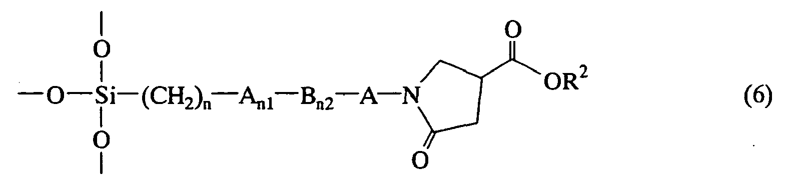 wherein R⁴ represents a monovalent hydrocarbon group, and R², n, n1 and n2, respectively, have the same meanings as defined before.

Accordingly, the invention provides an organopolysiloxane compound and an amidoamine compound defined below.
[1] An organopolysiloxane compound having a weight average molecular weight of 300 to 200,000 and having per molecule at least one organic group represented by the following formula (1) and at least one (R¹SiO_{3/2}) unit, wherein R¹ represents a hydrogen atom, or an organic group selected from an alkyl group, a cycloalkyl group, a fluorine-substituted alkyl group, an aryl group, an aralkyl group, an organooxy group and a group represented by the following formula (1), and at least one R¹ is a group represented by the formula (1): 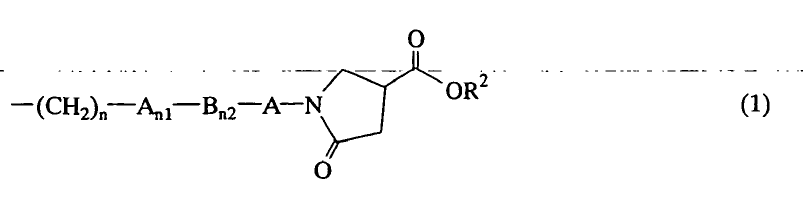 wherein R² represents a hydrogen atom, an alkyl group having 1 to 6 carbon atoms, or an alkali metal, A independently represents a linear or branched alkylene group having 1 to 12 carbon atoms, B represents -NR³-, sulfur or oxygen atom, in which R³ represents a hydrogen atom or an alkyl group having 1 to 6 carbon atoms, n is 0 or 2, n1 is 0 or 1, and n2 is 0 or 1 provided that when n is 0 and n2 is 1, n1 is 1, when n is 2 and n2 is 1, n1 is 0 or 1, and when n is 2 and n2 is 0, n1 is 0.
[2] The organopolysiloxane compound as defined in [1], wherein said organopolysiloxane compound is represented by the following formula (2):

   (R⁴₃SiO_{1/2})ₐ(R⁴₂SiO)_{b}(R¹SiO_{3/2})_{c}(SiO₂)_{d} (2)

   wherein R¹ has the same meaning as defined above, R⁴ independently represents a hydrogen atom or an organic group selected from an alkyl group, a cycloalkyl group, a fluorine-substituted alkyl group, an aryl group, an aralkyl group, an organooxy group and a group represented by the formula (1), a is 3 to 50, b is 0 to 2,000, c is 1 to 30 and d is 0 to 30 provided that c+d is 1 or over.
[3] The organopolysiloxane compound as defined in [1] or [2], wherein said organopolysiloxane compound comprises at least one (R¹SiO_{3/2}) unit and at least one R¹ is a group represented by the formula (1).
[4] An amidoamine compound obtained by the reaction between the group of the formula (1) of the organopolysiloxane compound defined in any one of claims [1] to [3] and an amine compound represented by the following formula (3):

   HNR⁵(CH₂)ₙ₃NR⁶R⁷ (3)

   wherein R⁵ represents a hydrogen atom, an alkyl group, a hydroxyalkyl group, an alkenyl group, a cycloalkyl group or a polyoxyalkylene group, R⁶ and R⁷ may be the same or different and represent an alkyl group, a hydroxyalkyl group, a cycloalkyl group, a carboxyalkyl group or a polyoxyalkylene group, or R⁶ and R⁷ may form an N-heterocyclic ring along with the nitrogen atoms thereof, and n3 is an integer of 0 or over.

Further, the invention provides cosmetic preparations, particularly skin or hair cosmetic preparations, which comprise the organopolysiloxane compound of [1], [2] or [3]. The invention also provides cosmetic preparations formulated with a powder treated with the organopolysiloxane compound.

The novel organopolysiloxane compound of the invention has a siloxane branched structure and thus, effective surface treatment of fibers, hair, paper, inorganic fillers and the like can be performed. Especially, the organopolysiloxane compound of the invention has such a structure that polysiloxane chains extend, in three directions, from the silicon atom joined to the organic group including a pyrrolidonecarboxyl group. Hence, when this organopolysiloxane compound is formulated, cosmetic preparations capable of effective surface treatment of the hair or powder are obtained. In addition, there can be obtained cosmetic preparations which are free of greasiness and are good at makeup lasting performance.

### BRIEF DESCRIPTION OF DRAWING

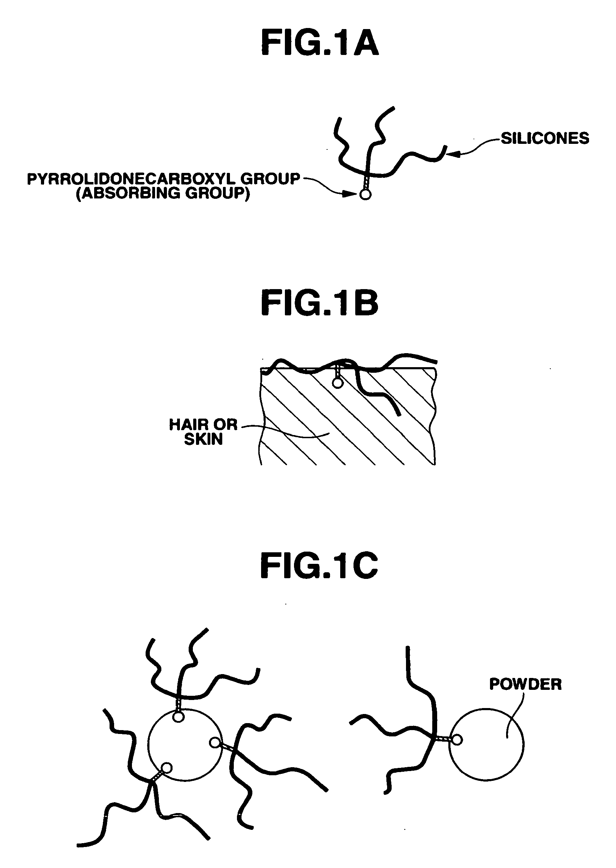

Figs. 1A to 1C are, respectively, an illustrative view wherein Fig. 1A schematically shows a characteristic portion of an organopolysiloxane of the invention, Fig. 1B schematically shows the organopolysiloxane applied onto the hair or skin, and Fig. 1C schematically shows powdery particles treated with the organopolysiloxane.

### DETAILED DESCRIPTIONS

### Organopolysiloxane

The organopolysiloxane of the invention has a weight average molecular weight of 300 to 200,000 and has at least one organic group represented by the formula (1) per molecule and also at least one (R¹SiO_{3/2}) unit wherein R¹ represents a hydrogen atom, or an organic group selected from an alkyl group, a cycloalkyl group, a fluorine-substituted alkyl group, an aryl group, an aralkyl group, an organooxy group and a group represented by the following formula (1), and at least one R¹ is a group represented by the formula (1): 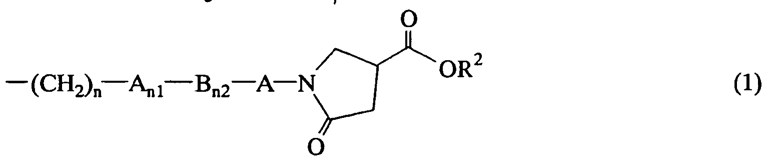 In the formulas, R¹ is a hydrogen atom, an alkyl group having 1 to 30 carbon atoms, preferably 1 to 16 carbon atoms, a cycloalkyl group having 3 to 6 carbon atoms, preferably 5 to 6 carbon atoms, a fluorine-substituted alkyl group wherein part (at least one) or all of the hydrogen atoms of the above-mentioned alkyl group are substituted with a fluorine atom, an aryl group having 6 to 20 carbon atoms, preferably 6 to 9 carbon atoms, an aralkyl group having 7 to 20 carbon atoms, preferably 7 to 10 carbon atoms, an organooxy group such as an alkoxy group having 1 to 20 carbon atoms, preferably 1 to 6 carbon atoms and an alkenyloxy group having 2 to 20 carbon atoms, preferably 2 to 6 carbon atoms, or a group represented by the above general formula (1). More specifically, R¹ is an alkyl group such as a methyl group, an ethyl group, a propyl group, a butyl group, a pentyl group, a hexyl group, a heptyl group, an octyl group, a nonyl group, a decyl group or the like, a cycloalkyl group such as a cyclopentyl group, a cyclohexyl group or the like, an aryl group such as a phenyl group, a tolyl group or the like, an aralkyl group such as a benzyl group, a phenetyl group or the like, an organooxy group such as an oleyloxy group, an allyloxy group or the like, a fluorine-substituted alkyl group such as a trifluoropropyl group, a heptadecafluorodecyl group or the like, or a group represented by the formula (1). Preferably, there is mentioned a hydrogen atom, a methyl group, an ethyl group, an octyl group, a nonyl group, a phenyl group, or a trifluoropropyl group.

R² represents a hydrogen atom, an alkyl group having 1 to 6 carbon atoms, an alkali metal such as Na, K, Li or the like. A's may be the same or different and represent a linear or branched alkylene group having 1 to 12 carbon atoms, preferably 1 to 4 carbon atoms, and B represents -NR³-, sulfur or oxygen atom wherein R³ represents a hydrogen atom or an alkyl group having 1 to 6 carbon atoms. n is 0 or 2, n1 is 0 or 1 and n2 is 0 or 1 provided that when n is 0 and n2 is 1, n1 is 1, when n is 2 and n2 is 1, n1 is 0 or 1, and when n is 2 and n2 is 0, n1 is 0.

Preferably, R² is a hydrogen atom or an alkyl group having 1 to 3 carbon atoms, A is an alkylene group having 1 to 3 carbon atoms, and B is -NH-. Preferred groups of the formula (1) are those group of the formulas (7) to (9) indicated below.

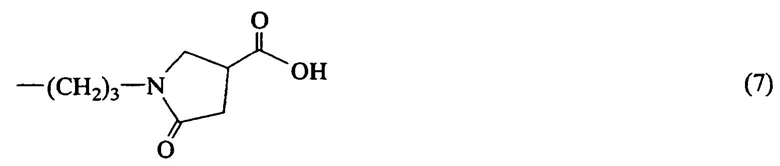

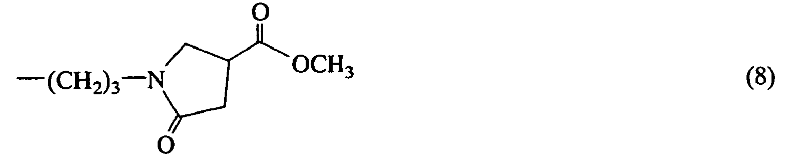

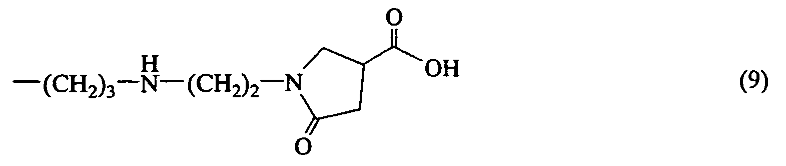

The organopolysiloxane compound of the invention Should have at least one of (R¹SiO_{3/2}) unit (hereinafter referred to as "T unit") represented by the following formula (10) and (SiO₂) unit (hereinafter referred to as "Q unit") represented by the following formula (11). The compound may have either the T unit alone or Q unit alone and should have T unit and/or Q unit. Although the T unit is represented by the following formula (10) and the Q unit is represented by the following formula (11) wherein the bonding sites extending from the oxygen atoms join to other Si atoms, respectively. Preferably, the total number of the T units and Q units is at 10 or below. More preferably, the T units are 1 to 5 in number and the Q units are 0 to 3.

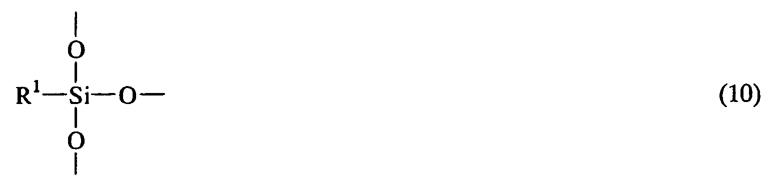

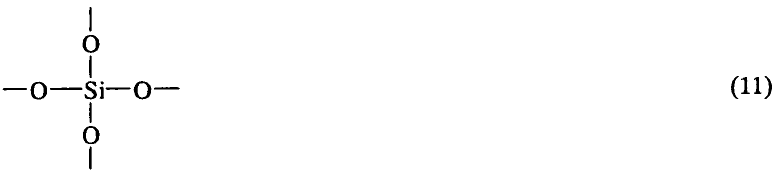

It is preferred that the organopolysiloxane compound of the invention contains one or more of the T units per molecule and at least one of R¹'s joining to the silicon atom in the T unit is an organic group represented by the general formula (1).

The weight average molecular weight of the organopolysiloxane compound of the invention determined by gel permeation chromatography (GPC) using polystyrene standards ranges 300 to 200,000, preferably 300 to 50,000 and more preferably 1,000 to 10,000.

The organopolysiloxane compound of the invention is preferably one represented by the following formula (2).

(R⁴₃SiO_{1/2})ₐ(R⁴₂SiO)_{b}(R¹SiO_{3/2})_{c}(SiO₂)_{d} (2)

The above formula (2) indicates the constituent units of the organopolysiloxane and the numbers thereof, not indicating the sequence of the units. In the formula, (R¹SiO_{3/2}) means the T unit and (SiO₂) means the Q unit.

In the above formula, R¹ has the same meaning as defined before.

R⁴ independently represents a hydrogen atom, an alkyl group, a cycloalkyl group, a fluorine-substituted alkyl group, an aryl group, an aralkyl group, an organooxy group, or a group represented by the foregoing formula (1) and the specific groups are those as mentioned with respect to R¹. Preferably, there is mentioned a methyl group, an ethyl group, an octyl group, a nonyl group, a phenyl group, a trifluoropropyl group, or a group represented by the formula (1).

In the formula (2), a is 3 to 50, b is 0 to 2,000, c is 1 to 30 and d is 0 to 30 provided that c+d is 1 or over. In this case, c indicating the number of the T units and d indicating the number of the Q units are preferably such that as indicated above, c+d is 10 or below, and it is more preferred that c is 1 to 5 and d is 0 to 3. Moreover, a and b are so appropriately selected that the weight average molecular weight is within a range of 300 to 200,000. Typically, a is 3 to 50, more typically 3 to 20 and b is 0 to 2,000, more typically 1 to 1,000.

The primary amino group-containing organopolysiloxane used in the invention can be prepared according to a method described in Silicone Handbook (pp. 165-166, published by Nikkan Kogyo Shimbun, Ltd.) wherein a terminal source and T and Q unit sources are appropriately selected from tris(trimethylsiloxy)methylsilane, tetrakis(tirmethylsiloxy)silane, tris(trimethylsiloxy)silylpropylamine, tris(trimethylsiloxy)silylpropylethylenediamine and the like and are polymerized in the presence of an alkali catalyst to prepare a primary amino group-containing branched organopolysiloxane.

The primary amino group-containing, branched organopolysiloxane can be readily, directly prepared in the same manner as described in JP-A 11-504665 wherein itaconic acid or an ester thereof is used in an amount of about 0.5 to 1.5 equivalents, preferably in an approximately stoichiometric amount, relative to a primary amine functional group and subjecting to reaction therebetween at high temperatures for a time sufficient to permit substantially all of itaconic acid or its ester to react with the primary amino functional group. Especially, when about 0.5 to 1.5 equivalents, preferably 0.9 to 1.1 equivalents of itaconic acid or its ester relative to the primary amino functional group is reacted, substantially all of the itaconic acid and all the primary amino functional group are reacted thereby forming an organopolysiloxane compound having at least one pyrrolidonecarboxyl functional group and/or an ester thereof.

In this case, itaconic acid or its ester used in the invention should preferably be one represented by the following formula (12):

CH₂=C(COOR⁸)CH₂COOR⁸ (12)

wherein R⁸ independently represents a hydrogen atom or an alkyl group having 1 to 6 carbon atoms.

The reaction can be carried out in a solvent-free condition or in a solvent such as of an alcohol, a hydrocarbon, a chlorinated hydrocarbon or the like preferably at about 90 to about 130°C. The reaction readily proceeds wherein the complete reaction between itaconic acid or its ester and the primary amino functional group occurs in about 1 to about 5 hours. The completion of the reaction can be determined by analysis of an amine number and an acid value in a usual manner and the measurement of discharged water and/or alcohol.

The organopolysiloxane compound of the invention may also be readily prepared by hydrosilylation reaction. The SiH group-containing organopolysiloxanes used herein can be prepared by the known methods. In the method, the organopolysiloxane is prepared by using, as the T unit and Q unit sources, tris(trimethylsiloxy)methylsilane, trimethoxymethylsilane, hexyltrimethoxysilane, octyltriethoxysilane, tetrakis(trimethylsiloxy)silane, tetramethoxysilane, tetraethoxysilane and the like, followed by polymerization along with hexamethylcyclotrisiloxane, octamethylcyclotetrasiloxane, decamethylcyclopentasiloxane and, if desired, hexamethyldisiloxane serving as a terminal end source in the presence of an acid catalyst.

In such a hydrosilylation reaction, there may be adopted a process wherein the organopolysiloxane obtained above as having one or two or more of hydride substituent group (SiH group) on the silicon chain is added to a pyrrolidone ring having an N-alkenylcarboalkoxy group, followed by reaction in the presence of a noble metal (group VIII metal) catalyst, preferably in the presence of a soluble platinum catalyst, at high temperatures (65 to 130°C) for a time sufficient to permit the reaction between all of the N-alkenylcarboalkoxy group-containing pyrrolidone and the hydride group (SiH group). The above-mentioned N-alkenylcarboalkoxy group-containing pyrrolidone reactant may further contain an N-allyl group or an olefinyl group having three or four carbon atoms, which may contain at least one hetero atom. The reaction can be carried out under solvent-free conditions or in an inert solvent such as of toluene, benzene, chlorobenzene, heptane or the like. In this case, it is preferred to react about 0.5 to 1 equivalent, preferably about 0.9 to 1.1 equivalents of the N-alkenylpyrrolidone group relative to the hydride group (SiH group) with the organopolysiloxane. In such a reaction, substantially all of the N-alkenylcarboalkoxy group-containing pyrrolidone groups and all of the hydride groups are preferably reacted. Preferred platinum catalysts include solubilized platinum compounds, or a platinum metal on an inert carrier such as alumina, activated carbon or the like.

### Amidoamine compound

The organopolysiloxane compound of the invention has at least one group represented by the following formula (1).

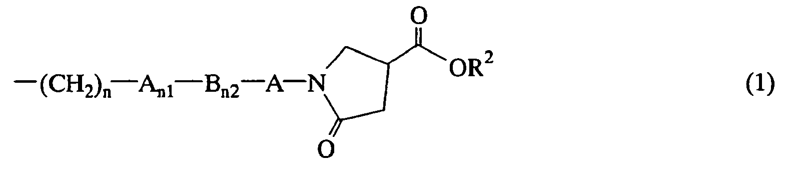

Accordingly, when the group of the organopolysiloxane compound of the formula (1) and the amine compound represented by the following formula (3) are reacted, there can be obtained an amidoamine compound having a group represented by the following formula (13) through dehydration, de-alcoholization and R²OH-removing reactions

HNR₅(CH₂)ₙ₃NR⁶R⁷ (3)

wherein R⁵ represents a hydrogen atom, an alkyl group, a hydroxyalkyl group, an alkenyl group, a cycloalkyl group or a polyoxyalkylene group, R⁶ and R⁷ may be the same or different and represent an alkyl group, a hydroxyalkyl group, a cycloalkyl group, a carboxyalkyl group or a polyoxyalkylene group, or R⁶ and R⁷ may form an N-heterocyclic ring along with the nitrogen atoms bound thereto, and n3 is an integer of 0 or over, 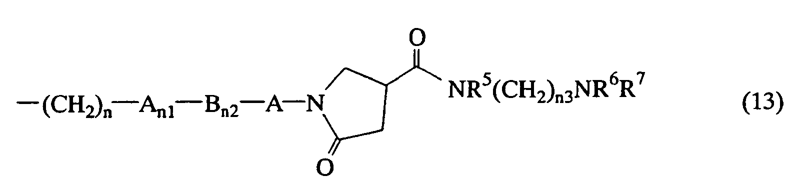 wherein R⁵ represents a hydrogen atom, an alkyl or hydroxyalkyl group having 1 to 6 carbon atoms, a cycloalkyl group having 3 to 6 carbon atoms, an alkenyl group having 2 to 6 carbon atoms, or a polyoxyalkylene group whose alkylene moiety has 1 to 6 carbon atoms, R⁶ and R⁷ may be the same or different and represent an alkyl or hydroxyalkyl group having 1 to 6 carbon atoms, a cycloalkyl group having 3 to 6 carbon atoms, a carboxyalkyl group whose alkyl moiety has 3 to 6 carbon atoms, or a polyoxyalkylene group whose alkylene moiety has 1 to 6 carbon atoms, or R⁶ and R⁷ may join to form an N-heterocyclic ring along with the nitrogen atoms bound thereto, e.g. a piperidine group, a pyrrole group or the like, and n3 is an integer of 0 or over, preferably 2 to 8.

The reactant ratio between the amine reactant and the carboxyl reactant is preferably at 1:1 by mole, and may be changed within a range of the ratio of 1:0.5 to 1:1.2. The reaction can be carried out under solvent-free conditions or in an inert solvent such as of xylene, toluene, chlorobenzene or the like. The reaction temperature is preferably at 100 to 150°C, and the reaction time is generally at 3 to 8 hours.

### Cosmetic preparation

When the organopolysiloxane compound of the invention is applied such as onto the skin or the like, greasiness is reduced and a dry feel is imparted, from which it can be used by formulation in cosmetic preparations. The compound may be formulated in cosmetic preparations as it is or after treating a variety of powders with the organopolysiloxane.

In more details, the invention provides cosmetic preparations containing the above-defined polyorganopolysiloxane compound according to the present invention. For this purpose, it is preferred to contain, as the organopolysiloxane compound, one that has a weight average molecular weight of 300 to 200,000 and has, per molecule, at least one (R¹SiO_{3/2}) unit wherein R¹ is an organic group represented by the foregoing formula (1). The group of the formula (1) preferably includes those groups of the afore-indicated formulas (7) to (9). Moreover, the specific examples of the organopolysiloxane compound include those compounds of the afore-indicated formula (2). For the cosmetic preparations, there are favorably used the compounds of the formula (2) wherein c is at 1 to 10, preferably at 1 to 3, and d is at 0 to 10, preferably at 1 to 5 provided that a and b are, respectively, as defined before.

The organopolysiloxane compound of the invention can be formulated in many cosmetic preparations as it is or after treatment of a variety of powders with this organopolysiloxane.

In the case, the compound may be formulated in the cosmetic preparations including skin cosmetics and hair cosmetics, e.g. a sunscreen agent, an milky lotion, a hair cream, a makeup remover, a hair style remover, a make remover, a rouge, a lip rouge, an eyeliner, an eye shadow, a sun-cut cream, a sun tan cream, a foundation, a moisturizing cream, a hand cream, a beauty essence, a perspiration deodorant, a cleansing cream, a hair treatment, a pack cosmetic, a deodorant and the like. The cosmetic preparations may take any form of solid, powder, gel, cream, liquid, O/W emulsion, O/W/O emulsion, W/O/W emulsion and the like.

When the organopolysiloxane is formulated in cosmetic preparations, the amount is varied depending on the type of cosmetic preparation, and it is preferred from the standpoint of showing the effect of the organopolysiloxane to formulate 1 to 50% by weight, especially 1 to 25% by weight, in the cosmetic preparation.

As will be seen from the afore-indicated formula (5), the organopolysiloxane compound of the invention has such a structure, as shown in Fig. 1A, that polysiloxane chains (silicone chains) extend in three directions from the silicon atom to which the pyrrolidonecarboxyl group-containing organic group joins. When this is applied to the hair or skin, the pyrrolidonecarboxyl group serving as an adsorption group adsorbs on the hair or skin as shown in Fig. 1B, whereas the silicone chains serving as a hydrophobic group are in a state of reliably covering the hair or skin, thereby reducing greasiness and imparting a dry feel. As shown in Fig. 1C, when powder is treated with the organopolysiloxane compound of the invention, mutual contact or coagulation of powdery particles lowers.

In the practice of the invention, when powder is treated with the organopolysiloxane compound, powders used are not critical with respect to the shape thereof (which may be spherical, needle-like, spindle-shaped, fan-like, or plate-like), particle size (which may be of fumed-like particles as in fumed silica, fine particles, a pigment grade or the like) and a particulate structure (which may be porous or non-porous) so far as they are used in ordinary cosmetic preparations. For instance, mention is made of inorganic powders, organic powders, surface active metal salt powders, colored pigments, pearl pigments, metal powder pigments, natural dyestuffs and the like.

Examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, white mica, synthetic mica, bronze mica, red mica, black mica, lepidolite, silicic acid, anhydrous silicic acid, aluminum silicate, magnesium silicate, aluminum magnesium silicate, calcium silicate, barium silicate, strontium silicate, metal tungstates, hydroxyapatite, vermiculite, Heidilite, bentonite, montmorillonite, hectorite, zeolite, ceramic powder, dicalcium phosphate, alumina, aluminum hydroxide, boron nitride, boron nitride, silica, glass, silylated silica and the like.

Examples of the organic powder include polyamide powder, polyacrylic acid/acrylic ester powder, polyester powder, polyethylene powder, polypropylene powder, polystyrene powder, polyurethane powder, benzoguanamine powder, polymethylbenzoguanamine powder, tetrafluoroethylene powder, polymethyl methacrylate powder, cellulose powder, silk powder, nylon powder such as 6 nylon or 12 nylon powder, crosslinked silicone fine powder having a structure of crosslinked dimethylsilicone, fine powder of polymethylsilsesquioxane, fine powder of crosslinked spherical organopolysiloxane rubber covered, on the surface thereof, with polymethylsilsesquioxane particles, hydrophobicized silica, powders of styrene/acrylic acid resin, divinylbenzene/styrene copolymer, vinyl resin, urea resin, phenolic resin, fluorine resin, silicon resin, acrylic acid, melamine resin, epoxy resin, polycarbonate resin and microcrystalline fibers, starch powder, fatty acid starch derivative powder, lauroyl lysine and the like.

Examples of the surface active metal salt powder (metallic soap) include zinc stearate, aluminum stearate, calcium stearate, magnesium stearate, zinc myristate, magnesium myristate, zinc cetyl phosphate, calcium cetyl phosphate, zinc sodium cetyl phosphate, zinc palmitate, aluminum palmitate, zinc laurate, and the like. Examples of the colored pigment include inorganic red pigments such as iron oxide, iron hydroxide, iron titanate and the like, inorganic brown pigments such as γ-iron oxide and the like, inorganic yellow pigments such as yellow iron oxide, yellow orcher and the like, inorganic black pigments such as black iron oxide, carbon black and the like, inorganic purple pigments such as manganese violet, chromium hydroxide, chromium oxide, cobalt oxide, cobalt titanate and the like, inorganic blue pigments such as iron blue, ultramarine blue and the like, laked tar dyestuff, laked natural dyestuff, synthetic resin powders making use of composites of these powders, and the like.

Examples of the pearl pigment include titanium oxide-coated isinglass, titanium oxide-coated mica, bismuth oxychloride, titanium oxide-coated bismuth oxychloride, titanium oxide-coated talc, argentine, titanium oxide-coated colored mica and the like. Examples of the metallic powder pigment include aluminum powder, cupper powder, stainless powder and the like. Examples of the tar dye include powders selected from Red Color No. 3, Red Color No. 104, Red Color No. 106, Red Color No. 201, Red Color No. 202, Red Color No. 204, Red Color No. 205, Red Color No. 220, Red Color No. 226, Red Color No. 227, Red Color No. 228, Red Color No. 230, Red Color No. 401, Red Color No. 505, Yellow Color No. 4, Yellow Color No. 5, Yellow Color No. 202, Yellow Color No. 203, Yellow Color No. 204, Yellow Color No. 401, Blue Color No. 1, Blue Color No. 201, Blue Color No. 404, Green Color No. 3, Green Color No. 201, Green Color No. 204, Green Color No. 205, Orange Color No. 201, Orange Color No. 203, Orange Color No. 204, Orange Color No. 206, Orange Color No. 207 and the like. Examples of the natural dye include powders selected from carminic acid, laccaic acid, carthamin, brazilin, crosin and the like. Mention is further made of powders capable of absorbing and scattering UV light such as fine-grain titanium oxide, fine-grain iron-containing titanium oxide, fine-grain zinc oxide, fine-grain cerium oxide and composite products thereof.

Among the powders mentioned above, titanium oxide, zinc oxide, mica, sericite, talc and kaolin are preferred for cosmetic purposes.

For the treatment of these powders, the organopolysiloxane compound of the invention is provided as a powder treating agent and is used in an amount of 0.1 to 30 parts by weight, preferably 0.5 to 10 parts by weight, relative to 100 parts by weight of the powder.

The powder treatment may be carried out by any of known ones and mention is made, for example, of the following methods.
1. A method wherein after mixing a powder and a powder treating agent, a powdering device such as a ball mill, a beads mill, a jet mill, a high pressure homogenizer or the like is used for surface treatment.
2. A treating method wherein a powder treating agent is dissolved or dispersed in a solvent and a powder is dispersed in the solution or dispersion to permit adsorption on the powder surface, followed by drying and baking.
3. A treating method wherein a powder is dispersed in an aqueous medium, to which a powder treating agent or its emulsion with an aqueous solvent to permit adsorption on the surface, followed by drying and baking.

The solvent used may be a hydrocarbon solvent, e.g. toluene. For the aqueous solvent, a mixture of water and an alcohol may be used. The powder treating agent of the invention is dissolved, dispersed or emulsified in a solvent in such an amount defined above relative to 100 parts by weight of the powder. Although depending on the type of solvent used, the baking is carried out by heating at 100 to 180°C for 1 to 5 hours.

In the invention, the thus surface-treated powder may be formulated as a powder dispersion obtained by dispersing the powder in an oil. The oil may be a hydrocarbon oil, an ester oil, a silicone oil or the like.

The hydrocarbon oil includes ozocerite, α-olefin oligomer, light isoparaffin, isododecane, isohexadecane, light liquid isoparaffin, squalane, synthetic squalane, plant squalane, squalane, ceresin and the like, of which isododecane and isohexadecane are preferred.

Examples of the ester oil include diisobutyl adipate, 2-hexyldecyl adipate, di-2-heptylundecyl adipate, N-alkylglycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dioctanoate, neopentyl glycol dicapriate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isononyl isonanoate, isotridecyl isonanoate, isopropyl palmitate, 2-ethylhexyl palmitate, 2-hexyldecyl palmitate, 2-heptylundecyl palmitate, choresteryl 12-hydroxystearate, dipentaerythritol fatty acid ester, isopropyl myristate, octyldodecyl myristate, 2-hexyldecyl myristate, myristyl myristate, hexyldecyl dimethyloctanoate, ethyl laurate, hexyl laurate, N-lauroyl-L-glutamic acid-2-octyldodecyl ester, lauroylsarcosine isopropyl ester, diisostearyl malate and the like. Examples of the glyceride oil include acetoglyceryl, glyceryl triisooctanoate, glyceryl triisostearate, glyceryl triisopalmitate, glyceryl tribehenate, glyceryl monostearate, glyceryl di-2-heptylundecanoate, glyceryl trimyristate, diglyceryl myristate stearate and the like. Preferably, there is mentioned isocetyl isostearate, cetyl octanoate and isononyl isononanoate.

As a silicone oil, mention is made of low to high viscosity, linear or branched organopolysiloxanes such as dimethylpolysiloxane, tristrimethylsiloxymethylsilane, capryl methicone, phenyl trimethicone, tetrakis(trimethylsiloxy)silane, methylphenyl polysiloxane, methylhexyl polysiloxane, methylhydrogenpolysiloxane, dimethylsiloxane/methylphenyl siloxane copolymer and the like, cyclic organopolysiloxanes such as octamethylcyclotetrasiloxane, decamethylcyclopentasiloxane, dodecamethylcyclohexasiloxane, tetramethyltetrahdyrogencyclotetrasiloxane, tetramethyltetraphenylcyclotetrasiloxane and the like, silicone rubbers such as amino-modified organopolysiloxane, pyrrolidone-modified organopolysiloxane, gum-like dimethylpolysiloxane of a high degree of polymerization, gum-like amino-modified organopolysiloxane, gum-like dimethylsiloxane/ethyl phenyl siloxane copolymer and the like, and silicone oils such as a cyclic organopolysiloxane solution such as of silicone gum or rubber, trimethylsiloxysilicic acid, a cyclic siloxane solution of trimethylsiloxysilicic acid, a higher alkoxy-modified silicone such as stearoxy silicone, a higher fatty acid-modified silicone, an alkyl-modified silicone, a long-chain alkyl-modified silicone, an amino acid-modified silicone, a fluorine-modified silicone, a silicone resin, a dissolved matter of a silicone resin and the like. Preferably, there are mentioned cyclic organopolysiloxanes such as octamethylcyclohexasiloxane, decamethylcyclopentasiloxane, dodecamethylcyclohexasiloxane, tetramethyltetrahydrogen cyclotetrasiloxane, tetramethyltetraphenyl cyclotetrasiloxane and the like.

For the preparation of a dispersion, 5 to 70 parts by weight, preferably 20 to 60 parts by weight of a powder, is dispersed in 30 to 95 parts by weight, preferably 40 to 80 parts by weight, of an oil. The dispersion can be made by a known means, e.g. a beads mill.

The powder set out above can be used in a variety of cosmetic preparations and is conveniently employed for cosmetics for external applications, especially, to the skin or hair, e.g. skin care products, makeup products, hair care products, antiperspirant products, UV protecting products and the like. The powder can be formulated in an amount of 0.1 to 99% by weight of the total of a cosmetic product although depending on the type and preparation thereof. The powder may be preliminarily provided in the form of a powder dispersion and subsequently formulated in cosmetic preparations.

The cosmetic preparations according to the invention may further comprise, in addition to the organopolysiloxane or the powder treated with the organopolysiloxane, a variety of components ordinarily employed in cosmetic preparations including, for example, A) oils, B) water, C) alcoholic hydroxyl group-containing compounds, D) water-soluble or swelling polymers, E) powders other than those powders of the invention and colorants, F) surfactants, G) silicone resins, H) compositions comprising crosslinked organopolysiloxanes and oils kept liquid at room temperature, I) silicone waxes, and other additives.

The oils A) may be any of solid, semi-solid and liquid, for which there can be used, for example, natural animal and plant oils and fats and semi-synthetic oils and fats, hydrocarbon oils, higher alcohols, ester oils, silicone oils, and fluorine-based oils. Of these, hydrocarbon oils, ester oils and silicone oils may be the same as those set forth with respect to the dispersion.

The natural animal and plant oils and fats, and semi-synthetic oils and fats include avocado oil, linseed oil, almond oil, plant hopper wax, perilla oil, olive oil, cacao oil, kapok oil, torreya oil, carnauba wax, liver oil, candelilla wax, refined candelilla wax, beef tallow, beef leg tallow, beef bone tallow, hardened beef tallow, apricot kernel oil, whale oil, hardened oil, wheat germ oil, sesame oil, rice germ oil, rice bran oil, sugar cane wax, sasanqua oil, sunflower oil, shea butter, China wood oil, sinnamon oil, jojoba wax, squalane, shellac wax, turtle oil, soybean oil, tea seed oil, camellia oil, evening primrose oil, corn oil, lard, canola oil, Japan wood oil, bran wax, horse fat, persic oil, palm oil, palm kernel oil, castor oil, hardened castor oil, castor oil fatty acid methyl ester, sunflower oil, grape seed oil, bayberry wax, jojoba oil, macadamia nut oil, beeswax, mink oil, meadowform seed oil, cotton seed oil, cotton wax, sumac wax, sumac kernel oil, montan wax, palm oil, hardened palm oil, (tri)palm oil fatty acid glyceride, sheep fat, peanut oil, lanolin, liquid lanolin, reduced lanolin, lanolin alcohol, hardened lanolin, lanolin acetate, acetylated lanolin alcohol, isopropyl lanolate, POE lanolin alcohol ether, POE lanolin alcohol acetate, polyethylene glycol lanolate, POE hydrogenated lanolin alcohol ether, egg yolk oil and the like. It will be noted that POE means polyoxyethylene.

The higher alcohols include lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyletetradecinol, cholesterol, phytosterol, POE cholesterol ether, monostearyl glycerine ether (batyl alcohol), monooleyl glyceryl ether (selachyl alcohol), and the like.

The amount of the oil A) depends on the type of preparation and may be within a range of 1 to 98% by weight of the total cosmetic.

The amount of water B) depends on the type of preparation and may be within a range of 1 to 95% by weight of the total cosmetic.

The compound C) having an alcoholic hydroxyl group includes a lower alcohol such as ethanol, isopropanol or the like, a sugar alcohol such as sorbitol, maltose or the like, a sterol such as cholesterol, sitosterol, phytosterol, lanosterol or the like, and a polyhydric alcohol such as butylene glycol, propylene glycol, dibutylene glycol, pentylene glycol or the like. The formulation amount may be within a range of 0.1 to 98% by weight of the total cosmetic preparation.

The water-soluble or swelling polymers D) include plant polymers such as gum arabic, tragacanth, galactan, locust bean gum, guar gum, gum karaya, carageenan, pectin, agar-agar, quince seed (marmelo), starches (such as of rice, corn, potato, wheat and the like), algecolloid, trant gum, locust bean gum and the like, microorganism polymers such as xanthan gum, dextran, succinoglucane, pullulan and the like, animal polymers such as collagen, casein, albumin, gelatin and the like, starch polymers such as carboxymethyl starch, methylhydroxypropyl starch and the like, cellulose polymers such as methylcellulose, ethylcellulose, methylhydroxypropylcellulose, carboxymethylcellulose, hydroxymethylcellulose, hydroxypropylcellulose, nitrocellulose, sodium cellulose sulfate, sodium carboxymethylcellulose, crystalline cellulose, cellulose powder and the like, alginate polymers such as sodium alginate, propylene glycol alginate ester and the like, vinyl polymers such as polyvinyl methyl ether, carboxy vinyl polymer and the like, polyoxyethylene-based polymers, polyoxyethylene/polyoxypropylene copolymer-based polymers, acrylic polymers such as sodium polyacrylate, polyethylene acrylate, polyacrylamide, acryloyl dimethyl taurate copolymer and the like, other types of synthetic water-soluble polymers such as polyethyleneimine, cationic polymer and the like, and inorganic water-soluble polymers such as bentonite, aluminum magnesium silicate, montmorillonite, beidellite, nontronite, saponite, hectlite, silicic anhydride and the like. These water-soluble polymers may contain film-forming agents such as polyvinyl alcohol, polyvinyl pyrrolidone and the like. The amount is preferably within a range of 0.1 to 25% by weight of the total cosmetic preparation.

The powders and/or colorants E) include a variety of powders mentioned above. These powders may be used in the form of composite powders or treated with ordinary oils, silicone oils, fluorine compounds, surfactants and the like, or may be treated with a hydrolysable silyl group or an alkyl group having a hydrogen atom directly bonded to a silicon atom within a range not impeding the effects of the invention. In addition, there may be used, if necessary, one or more of a linear or branched organopolysiloxane having a hydrolysable silyl group or a hydrogen atom directly bonded to a silicon atom, a linear or branched organopolysiloxane that has a hydrolysable silyl group or a hydrogen atom directly bonded to a silicon atom and co-modified with a long alkyl, a linear or branched organopolysiloxane that has a hydrolysable silyl group or a hydrogen atom directly bonded to a silicon atom and co-modified with polyoxyalkylene, an acryl-silicone copolymer having a hydrolysable silyl group or a hydrogen atom directly bonded to a silicon atom, and the like. The amount of these powders is within a range not impeding the effect of formulating the powder of the invention.

The surfactants F) include anionic, cationic, nonionic and amphoteric surfactants and are not critical in type, and any of surfactants employed in ordinary cosmetic preparaions may be used.

The anionic surfactants include fatty acid soaps such as sodium stearate, triethanolamine palmitate and the like, alkyl ether carboxylic acids and salts thereof, condensate salts of amino acids and fatty acids, alkanesulfonates, alkenesulfonates, sulfonates of fatty acid esters, sulfonates of fatty acid amides, formalin condensate-based sulfonates, alkylsulfuric acid ester salts, secondary higher alcohol sulfuric acid ester salts, alkyl and allyl ether sulfuric acid ester salts, sulfuric acid ester salts of fatty acid esters, sulfuric acid ester salts of fatty acid alkylolamides, sulfuric acid ester salts such as of roto oil, alkylphosphates, ether phosphates, alkyl allyl ether phosphates, amidophosphates, N-acyl lactates, N-acyl sarcosinate, and N-acylamino acid surfactants. The cationic surfactants include alkylamine salts, amine salts such as polyamine and amino alcohol fatty acid derivatives, alkyl quaternary ammonium salts, aromatic quaternary ammonium salts, pyridinium salts, imidazolinium salts and the like.

The nonionic surfactants include sorbitan fatty acid esters, glycerine fatty acid esters, polyglycerine fatty acid esters, propylene glycol fatty acid esters, polyethylene glycol fatty acid esters, saccharose fatty acid esters, methyl glucoside fatty acid esters, alkyl polyglucosides, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerine fatty acid esters, polyoxyethylene propylene glycol fatty acid esters, polyoxyethylene castor oil, polyoxyethylene hardened castor oil, polyoxyethylene phytostanol ether, polyoxyethylene phytosterol ether, polyoxyethylene cholestarol ether, polyoxyethylene cholesteryl ether, linear or branched polyoxyalkylene-modified organopolysiloxanes, linear or branched polyoxyalkylene/alkyl co-modified organopolysiloxanes, linear or branched polyglycerine-modified organopolysiloxanes, linear or branched polyglycerine/alkyl-modified organopolysiloxanes, alkanolamides, sugar ethers, sugar amides and the like. The amphoteric surfactants include betaine, phosphatidylcholine, aminocarboxylates, imidazoline derivatives, amidoamine surfactants and the like.

Of these surfactants, it is preferred to use linear or branched organopolysiloxanes having a polyoxyalkylene chain or polyglycerine chain in the molecule, or linear or branched organopolysiloxanes having a long-chain alkyl group that has 6 to 20 carbon atoms. In these surfactants, it is preferred that the content of the hydrophilic polyoxyalkylene group or polyglycerine residue ranges 10 to 70% by weight in the molecule. The formulation amount is conveniently within a range of 0.1 to 20% by weight, preferably 0.2 to 10% by weight, of the total cosmetic preparation.

The silicone resin G) is preferably made of an acryl silicone resin in the form of an acryl/silicone graft or block copolymer. Alternatively, there may be used acryl silicone resins containing, in the molecule, at least one anionic member selected from a pyrrolidone moiety, a long-chain alkyl moiety, a polyoxyalkylene moiety, a fluoroalkyl moiety and an anionic moiety such as of a carboxylic acid. Additionally, preferred silicone resins include silicone network compounds made of resins constituted of R¹₃SiO_{0.5} unit and SiO₂ unit, resins constituted of R¹₃SiO_{0.5}, R¹₂SiO unit and SiO₂ unit, resins constituted of R¹₃SiO_{0.5} and R¹SiO_{1.5}, resins constituted of R¹₃SiO_{0.5}, R¹₂SiO and R¹SiO_{1.5}, and resins constituted of R¹₃SiO_{0.5}, R¹₂SiO, R¹SiO_{1.5} and SiO₂ unit. Still alternatively, there may be used silicone network compounds containing in the molecule at least one member selected from a pyrrolidone moiety, a long-chain alkyl group, a polyoxyalkylene moiety, a fluoroalkyl moiety and an amino moiety. The amount of the silicone resin such as an acryl silicone resin or a silicone network compound is preferably at 0.1 to 20% by weight, more preferably at 1 to 10% by weight, relative to the total amount of a cosmetic preparation.

In a composition H) comprised of a crosslinking organopolysiloxane and an oil kept liquid at room temperature, the crosslinking organopolysiloxane should preferably be swollen with a liquid oil as containing the liquid oil in an amount larger than its own weight. Usable liquid oils include liquid silicone oils, hydrocarbon oils, ester oils, natural animal and plant oils, semi-synthetic oils, fluorine-based oils and the like in the component A). Examples include low-viscosity silicone oils whose viscosity ranges 0.65 mm²/second (25°C) to 100.0 mm²/second (25°C), hydrocarbon oils such as liquid paraffin, squalane, isododecane, isohexadecane and the like, ester oils such as glyceride oils such as of trioctanoin, isotridecyl isononanoate, N-acyl glutamic acid ester, lauroyl sarcosine acid ester and the like, and natural animal and plant oils such as macadamia nut oil and the like. The crosslinking agent for the crosslinking organopolysiloxane should preferably have two or more reactive vinyl moieties in the molecule and allow reaction with the hydrogen atom directly bonded to a silicon atom, thereby forming a crosslinked structure. Those having two or more reactive vinyl moieties in the molecule include organopolysiloxanes having two or more reactive vinyl moieties in the molecule, polyoxyalkylenes having two or more allyl groups in the molecule, polyglycerines having two or more allyl groups in the molecules, α,ω-alkenyldienes, and the like.

Further, there may also be used crosslinking organopolysiloxanes containing in a crosslinked molecule at least one selected from the group consisting of a polyoxyalkylene moiety, a polyglycerine moiety, a long-chain alkyl moiety, an alkenyl moiety, an aryl moiety and a fluoroalkyl moiety. Where there is used a composition comprising this crosslinking organopolysiloxane and an oil kept liquid at room temperature, its amount is preferably at 0.1 to 80% by weight, more preferably at 1 to 50% by weight, of the total amount of a cosmetic preparation.

Silicone waxes I) include, for example, a silicone-modified olefin wax obtained by subjecting an unsaturated group-containing olefin wax made of an α-olefin and a diene and an organohydrogenpolysiloxane having one or more SiH bonds in the molecule to addition reaction therebetween. The α-olefins of the olefin wax preferably include ones having 2 to 12 carbon atoms such as ethylene, propylene, 1-butene, 1-hexene, 4-methyl-1-pentene and the like. The dienes preferably include butadiene, isoprene, 1,4-hexadiene, vinyl norbornene, ethylidenenorbornene, dicylcopentadiene and the like. A usable organohydrogenpolysiloxane having the SiH bonds may be one that has a linear or branched structure.

For other components or ingredients, mention is made of oil-soluble gelling agents, organo-modified clay minerals, resins, antiperspirants, UB absorbers, UV absorbing and scattering agents, humectants, preservatives, antibactericides, flavors, salts, antioxidants, pH adjusters, chelating agents, algefacients, anti-inflammatory agents, ingredients for skin beauty (bleachers, cell activators, improvers for skin roughness, blood circulation accelerators, skin astringents, antiseborrheic agents and the like), vitamins, amino acids, nucleic acids, hormones, clathrate compounds, solidifying agents for hair, and the like.

The oil-soluble gelling agent is one selected from a metallic soap such as aluminum stearate, magnesium stearate, zinc myristate or the like, an amino acid derivative such as N-lauroyl-L glutamic acid, α,γ-di-n-butylamine or the like, a dextrin fatty acid ester such as dextrin palmitic acid ester, dextrin stearic acid ester, dextrin 2-ethylhexanoic acid palmitic acid ester or the like, a saccharose fatty acid ester such as saccharose palmitic acid ester, saccharose stearic acid ester or the like, a fructo-oligosaccharide fatty acid ester such as fructo-oligosaccharide stearic acid ester, fructo-oligosaccharide 2-ethylheanoic acid or the like, a benzylidene derivative of sorbitol such as monobenzylidene sorbitol, dibenzylidene sorbitol or the like, and an organo-modified clay mineral such as dimethylbenzyldodecylammonium montmorillonite clay, dimethyldioctadecylammonium montmorillonite clay or the like.

The antiperspirant is one selected from aluminum chlorohydrate, aluminum chloride, aluminum sesqui-chlorohydrate, zirconium hydroxychloride, aluminum zirconium hydroxychloride, aluminum zirconium glycine complex and the like.

The UV absorber includes a benzoic acid UV absorber such as para-aminobenzoic acid or the like, an anthranilic acid UV absorber such as methyl anthranilate or the like, a salicyclic acid UV absorber such as methyl salicylate, octyl salicylate, trimethylcyclohexyl salicylate or the like, a cinnamic acid UV absorber such as paramethoxy octyl cinnamate or the like, a benzophenone UV absorber such as 2,4-dihydroxybenzophenone or the like, an urocanic acid UV absorber such as ethyl urocanate or the like, a dibenzoylmethane UV absorber such as 4-t-butyl-4'-methoxy-dibenzoulmethane or the like, phenylbenzimidazolesulfonic acid, a triazine derivative or the like. The UV absorbing and scattering agent includes powders capable of absorbing and scattering UV light such as fine-grain titanium oxide, fine-grain iron-containing titanium oxide, fine-grain zinc oxide, fine-grain cerium oxide and composite powders thereof. These powders capable of absorbing and scattering UV light may be used in the form of a dispersion obtained by preliminarily dispersing them in an oil.

The humectant includes glycerine, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate, polyoxyethylene methyl glycoside, polyoxypropylene methyl glucoside, egg yolk lecithin, soybean lecithin, phosphatidylcholine, phosphatidylethanolamine, phosphatidylserine, phosphatidylglycerol, phosphatidylinocitol, phosphosphingolipid or the like.

The antibacterial and preservative agent includes para-oxybenzoic acid alkyl ester, benzoic acid, sodium benzoate, sorbic acid, potassium sorbate, phenoxyethanol and the like, and the antibactericide includes benzoic acid, salicylic acid, phenolic acid, sorbic acid, p-oxybenzoic acid alkyl ester, p-chloro-m-cresol, hexachlorophene, benzalkonium chloride, chlorohexidine chloride, trichlorocarbanide, sensitizing dye, phenoxyethanol or the like.

The salts include inorganic salts, organic acid salts, amine salts and amino acid salts. Examples of the inorganic salt include sodium, potassium, magnesium, calcium, aluminum, zirconium, zinc and the like salts of inorganic acids such as hydrochloric acid, sulfuric acid, carbonic acid, nitric acid and the like. Examples of the organic acid salts include salts of organic acids such as acetic acid, dehydroacetic acid, citric acid, malic acid, succinic acid, ascorbic acid, stearic acid and the like. Examples of the amine salts and amino acid salts include salts of amines such as triethanolamine, and salts of amino acids such as glutamic acid. Besides, mention is made of salts of hyaluronic acid, chondroitin sulfuric acid and the like, aluminum zirconium glycine complex, and also of acid-alkali neutralized salts used in cosmetic formulations. The antioxidant includes tocopherol, p-t-butylphenol, butylhydroxyanisole, dibutylhydroxytoluene, phytic acid or the like. The pH adjuster includes lactic acid, citric acid, glycolic acid, succinic acid, tartaric acid, dl-malic acid, potassium carbonate, sodium hydrogencarbonate, ammonium hdyrogencarbonate or the like. The chelating agent includes alanine, sodium edetate, sodium polyphosphate, sodium methaphosphate, phosphoric acid or the like. The algefacient includes L-menthol, camphor or the like. The anti-inflammatory agent includes allantoin, glycyrrhizic acid and salts thereof, glycyrrhetinic acid and stearyl glycyrrhetinate, tranexamic acid, azulene or the like.

The ingredients for skin beauty include bleachers such as an placenta extract, alubmin, glutathione, saxifrage extract and the like, cell activators and skin roughness improvers such as royal jelly, sensitizing dyes, cholesterol derivatives, solcoseryl and the like, blood circulation accelerators such as nonylic acid vanillylamide, nicotinic acid benzyl ester, nicotinic acid β-butoxyethyl ester, capsaicin, zingerone, cantharis tincture, ichthammol, caffein, tannic acid, alpha-borneol, nicotinic acid tocopherol, inositol hexanicotinate, cyclandelate, cinnarizine, tolazoline, acetylcholine, verapamil, cepharanthin and γ-orizanol, skin astringents such as zinc oxide, tannic acid and the like, and antiseborrheic agents such as sulfur and thianthrol.

Examples of the vitamins include vitamin A's such as vitamin A oil, retinol, retinol acetate and retinol palmitate, vitamin B's including vitamin B2's such as riboflavin, riboflavin butyrate and flavin adenine nucleotide, vitamin B6's such as pyridoxine hydrochloride, pyridoxine dioctanoate and pyridoxine tripalmitate, vitamin B12 and its derivatives, and vitamin B15 and its derivatives, vitamin C's such as L-ascorbic acid, L-ascorbic acid dipalmitic acid ester, sodium L-ascorbic acid-2-sulfate and dipotassium L-ascorbic acid phosphoric acid diester, vitamin D's such as ergocalciferol, cholecalciferol and the like, vitamin E's such as α-tocopherol, β-tocopherol, γ-tocopherol, dl-α-tocopherol acetate, dl-α-tocopherol nicotinate, dl-α-tocopherol succinate, vitamin H, vitamin P, nicotinic acids such as nicotinic acid, benzyl nicotinate, nicotinic acid amide and the like, pantothenic acids such as calcium pantothenate, D-pantothenyl alcohol, pantothenyl ethyl ether and acetyl pantothenyl ethyl ether and the like, and biotin and the like.

Examples of the amino acids include glycine, valine, leucine, isoleucine, serine, threonine, phenylalanine, arginine, lysine, aspartic acid, glutamic acid, cystine, cysteine, methionine, tryptophan and the like. Examples of the nucleic acids include deoxyribonucleic acid and the like. Examples of the hormones include estradiol, ethenyl estradiol and the like.

The polymer compounds of hair fixation include amphoteric, anionic, cationic and nonionic polymer compounds. For this, there can be conveniently used polyvinylpyrrolidone compounds such as vinyl pyrrolidone/vinyl acetate copolymer and the like, acidic vinyl ether polymer compounds such as methyl vinyl ether/maleic anhydride alkyl half-ester copolymer and the like, acidic polyvinyl acetate polymers such as vinyl acetate/crotonic acid copolymer and the like, acidic acrylic polymer compounds such as (meth)acrylic acid/alkyl (meth)acrylate copolymers, (meth)acrylic acid/alkyl (meth)acrylate/alkylacrylamide copolymers and the like, amphoteric acryl polymer compounds such as N-methacryloylethyl-N,N-dimethylammonium α-N-methylcarboxybetaine/alkyl (meth)acrylate copolymer, hydroxypropyl (meth)acrylate/butylaminoethyl methacrylate/acrylic acid octyl amide copolymer, and the like. Alternatively, there may be conveniently used naturally occurring polymer compounds such as cellulose or derivatives thereof, and keratin and collagen or derivatives thereof, and the like.

The cosmetic preparations of the invention may take forms of powders, oils, water-in-oil emulsions, oil-in-water emulsions, non-aqueous emulsions, W/O/W, O/W/O and the like multiemulsions and the like. Alternatively, the cosmetic preparations may be in the form of liquid, milky lotion, cream, solid, paste, gel, powder, pressed matter, multi-layer, mousse, spray, stick, pencil and the like. Examples of the cosmetic preparations include skin care cosmetics such as a skin lotion, a milky lotion, cream, cleansing cream, pack, oil liquid, massage cosmetic, beauty essence, beauty oil, cleansing agent, deodorant, hand cream, lip cream, wrinkle concealer and the like, makeup cosmetics such as a foundation, makeup foundation, concealer, face powder, powder foundation, liquid foundation, cream foundation, oil-based foundation, rouge, eye shadow, mascara, eyeliner, eyeblow, lipstick and the like, hair cosmetics such as a shampoo, rinse, hair treatment, setting agent and the like, antiperspirants, and UV care cosmetics such as a sunscreen oil, sunscreen lotion, sunscreen cream and the like.

### EXAMPLES

The invention is described in more detail by way of Examples, which should not be construed as limiting the invention thereto. It will be noted that unless otherwise specified, "%" used herein is by mass and "parts" is by weight.

Examples 1 to 5 are not according to the invention.

### Example 1

44 parts of tris(trimethylsiloxy)methylsilane, 600 parts of octamethylcyclotetrasiloxane and 75 parts of aminopropylpolysiloxane represented by the following formula: 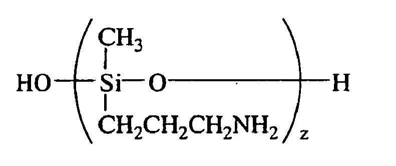 wherein z is an integer, were charged into a separable flask, to which 0.8 parts of 3% potassium siliconate was added, followed by agitation at 150°C for 5 hours. 1.5 parts of ethylene chlorohydrin was added to the resulting reaction mixture and agitated at 100°C for 1 hour for neutralization. Subsequently, unreacted silane and siloxane were distilled off under reduced pressure to obtain a branched organopolysiloxane represented by the following average compositional formula:

[(CH₃)₃SiO_{1/2}]₃[(CH₃)₂SiO]₅₇(CH₃)(CH₂CH₂CH₂NH₂)SiO]_{4.5}[CH₃SiO_{3/2}]₁.

This product was a colorless transparent liquid and had an amine equivalent of 1,066 g/mol.

350 parts of the thus obtained branched organopolysiloxane and 42 parts of itaconic acid were charged into a separable flask, followed by agitation at 150°C for 4 hours to distill off water. The resulting product was a light yellow transparent liquid wherein the CH₂CH₂CH₂NH₂ moiety in the above formula was substituted with a moiety of the following formula: 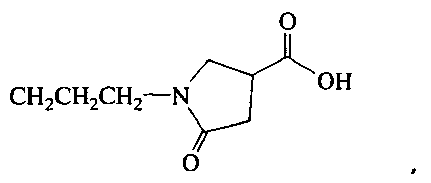 and had a carboxy equivalent of 1.299 g/mol. The results of measurement of GPC revealed that the weight average molecular weight was at 5,100. The ¹³C-NMR spectra of the thus obtained organopolysiloxane were shown in Table 1 below.

**Table 1**

| ¹³C-NMR (CDCl₃) | |
|---|---|
| 1.5 ppm | (Si-CH₃) |
| 12.3 ppm | (Si-CH₂-CH₂-CH₂) |
| 18.9 ppm | (Si-CH₂-CH₂-CH₂) |
| 46.7 ppm | (Si-CH₂-CH₂-CH₂) |
| 172.3 ppm | (N-CO) |
| 36.4 ppm | (CO-CH₂) |
| 45.4 ppm | (N-CH₂) |
| 34.5 ppm | (CH) |
| 178.3 ppm | (COOH) |

### Example 2

98 parts of tris(trimethylsiloxy)methylsilane, 347 parts of octamethylcyclotetrasiloxane and 55 parts of aminopropylpolysiloxane represented by the following formula: 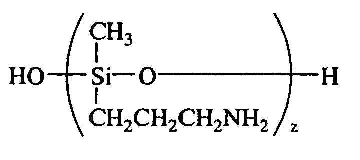 wherein z is an integer, were charged into a separable flask, to which 0.5 parts of 3% potassium siliconate was added, followed by agitation at 150°C for 5 hours. 1 part of ethylene chlorohydrin was added to the resulting reaction mixture and agitated at 100°C for 1 hour for neutralization. Subsequently, unreacted silane and siloxane were distilled off under reduced pressure to obtain a branched organopolysiloxane represented by the following average compositional formula:

[(CH₃)₃SiO_{1/2}]₃[(CH₃)₂SiO]₁₅[(CH₃)(CH₂CH₂CH₂NH₂)SiO]_{1.5}[CH₃SiO_{3/2}]₁ .

This product was a colorless transparent liquid and had an amine equivalent of 945 g/mol.

350 parts of the thus obtained branched organopolysiloxane and 14.7 parts of dimethyl itaconate were charged into a separable flask, followed by agitation at 140°C for 4 hours to distill off methanol. The resulting product was a yellow transparent liquid wherein the CH₂CH₂CH₂NH₂ moiety in the above formula was substituted with a moiety of the following formula: 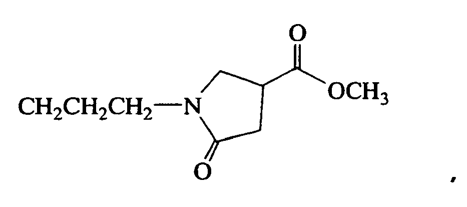 and an amine equivalent and a carboxy equivalent thereof were both at 0 g/mol. The results of measurement of GPC revealed that the weight average molecular weight was at 1,800. The ¹³C-NMR spectra of the thus obtained organopolysiloxane were shown in Table 2 below.

**Table 2**

| ¹³C-NMR (CDCl₃) | |
|---|---|
| 1.5 ppm | (Si-CH₃) |
| 11.3 ppm | (Si-CH₂-CH₂-CH₂) |
| 18.3 ppm | (Si-CH₂-CH₂-CH₂) |
| 45.3 ppm | (Si-CH₂-CH₂-CH₂) |
| 172.0 ppm | (N-CO) |
| 35.9 ppm | (CO-CH₂) |
| 45.2 ppm | (N-CH₂) |
| 34.1 ppm | (CH) |
| 178.0 ppm | (COOCH₃) |
| 52.0 ppm | (COOCH₃) |

### Example 3

35 parts of tetrakis(trimethylsiloxy) silane, 740 parts of octamethylcyclotetrasiloxane and 94 parts of methylaminopropylpolysiloxane represented by the following formula: 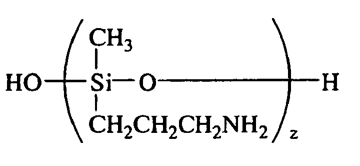 wherein z is an integer, were charged into a separable flask, to which 0.8 parts of 3% potassium siliconate was added, followed by agitation at 150°C for 5 hours. 1.5 parts of ethylene chlorohydrin was added to the resulting reaction mixture and agitated at 100°C for 1 hour for neutralization. Subsequently, unreacted silane and siloxane were distilled off under reduced pressure to obtain a branched organopolysiloxane represented by the following average compositional formula:

[(CH₃)₃SiO_{1/2}]₄[(CH₃)₂SiO]₁₀₀[(CH₃)(CH₂CH₂CH₂NH₂)SiO]₈[SiO₂]₁ .

This product was a colorless transparent liquid and had an amine equivalent of 1,120 g/mol.

300 parts of the thus obtained branched organopolysiloxane and 35 parts of itaconic acid were charged into a separable flask, followed by agitation at 140°C for 4 hours to distill off methanol. The resulting product was a yellow transparent liquid wherein the CH₂CH₂CH₂NH₂ moiety in the above formula was substituted with a moiety of the following formula: 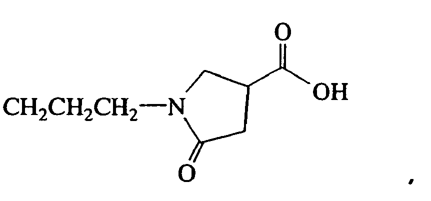 and had a carboxy equivalent of 1,250 g/mol. The results of measurement of GPC revealed that the weight average molecular weight was at 10,000. The ¹³C-NMR spectra of the thus obtained organopolysiloxane were shown in Table 3 below.

**Table 3**

| ¹³C-NMR (CDCl₃) | |
|---|---|
| 1.5 ppm | (Si-CH₃) |
| 12.3 ppm | (Si-CH₂-CH₂-CH₂) |
| 19.1 ppm | (Si-CH₂-CH₂-CH₂) |
| 46.7 ppm | (Si-CH₂-CH₂-CH₂) |
| 172.3 ppm | (N-CO) |
| 36.4 ppm | (CO-CH₂) |
| 45.4 ppm | (N-CH₂) |
| 34.5 ppm | (CH) |
| 178.8 ppm | (COOH) |

### Example 4

200 parts of the branched organopolysiloxane obtained in Example 1 and having a carboxy equivalent of 1,299 g/mol and 31 parts of dimethylaminopropylamine were charged into a separable flask and agitated at 150°C for 5 hours to distill off water. Subsequently, unreacted reactants were distilled off under reduced pressure and the resulting reaction product was a yellow transparent liquid wherein: 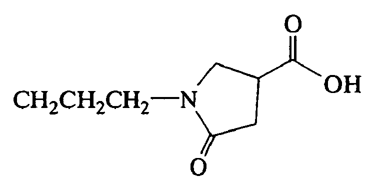 was replaced by 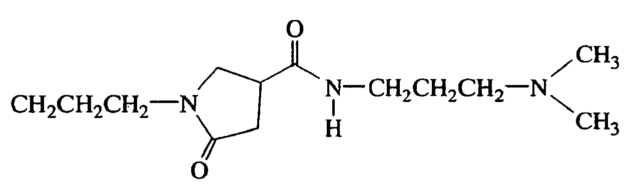 with an amine equivalent being at 1,370 g/mol. The results of measurement of GPC revealed that the weight average molecular weight was at 5,500. Moreover, ¹³C-NMR spectra of the obtained organopolysiloxane are shown in Table 4 below.

**Table 4**

| ¹³C-NMR (CDCl₃) | |
|---|---|
| 1.5 ppm | (Si-CH₃) |
| 12.3 ppm | (Si-CH₂-CH₂-CH₂) |
| 19.1 ppm | (Si-CH₂-CH₂-CH₂) |
| 46.7 ppm | (Si-CH₂-CH₂-CH₂) |
| 172.3 ppm | (N-CO) |
| 36.4 ppm | (CO-CH₂) |
| 45.4 ppm | (N-CH₂) |
| 34.5 ppm | (CH) |
| 174.8 ppm | (CH-CON) |
| 36.3 ppm | (N-CH₂-CH₂-CH₂-) |
| 22.3 ppm | (N-CH₂-CH₂-CH₂-) |
| 37.4 ppm | (N-CH₂-CH₂-CH₂-) |
| 43.0 ppm | (N-CH₃) |

### Example 5

32.4 parts of hexamethyldisiloxane, 55.2 parts of octyltriethoxysilane, 12.0 parts of tetramethylcyclotetrasiloxane and 222.0 parts of octamethylcyclotetrasiloxane were charged into a separable flask, to which 19 parts of concentrated sulfuric acid and 5.4 parts of water were added, followed by agitation at room temperature for 10 hours, washing with water and distilling off unreacted silane and siloxanes to obtain a branched organohydrogenpolysiloxane represented by the following average compositional formula:

[(CH₃)₃SiO_{1/2}]₄[(CH₃)₂SiO]₃₀[(CH₃)HSiO]₂[C₈H₁₇SiO_{3/2}]₂ .

This product was a colorless transparent liquid and had a kinetic viscosity of 58 MM²/s (25°C).

200 parts of this branched organohydrogenpolysiloxane and 100 parts of toluene were charged into a separable flask, to which 0.5 parts of a toluene solution of chloroplatinic acid complex (0.5% of platinum) was added. While agitating this mixture at 110°C, 23.4 parts of N-allyl-4-carbomethoxypyrrolidone represented by the following formula: 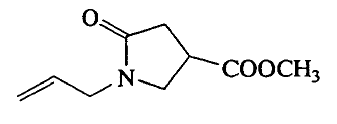 was gently dropped thereinto.

After agitation at 110°C for 5 hours, unreacted reactants were distilled off under reduced pressure to obtain a light yellow transparent liquid. As a result of NMR measurement, this reaction product has no SiH group. Hence, it was confirmed that (CH₃)HSiO in the above compositional formula was replaced by a moiety of the following formula: 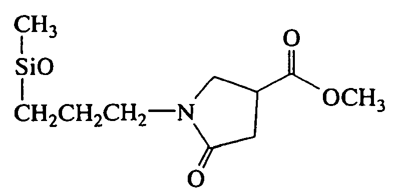 thereby completing the reaction. The results of measurement of GPC revealed that the product had a weight average molecular weight of 3,200. In addition, ¹³C-NMR spectra of the organopolysiloxane are shown in Table 5 below.

**Table 5**

| ¹³C-NMR (CDCl₃) | |
|---|---|
| 1.5 ppm | (Si-CH₃) |
| 11.8 ppm | (Si-CH₂-CH₂-CH₂) |
| 18.5 ppm | (Si-CH₂-CH₂-CH₂) |
| 44.7 ppm | (Si-CH₂-CH₂-CH₂) |
| 172.7 ppm | (N-CO) |
| 35.6 ppm | (CO-CH₂) |
| 45.2 ppm | (N-CH₂) |
| 34.1 ppm | (CH) |
| 178.0 ppm | (COCH₃) |
| 52.0 ppm | (COOCH₃) |
| 12.7-31.2 ppm | (Si-C₈H₁₇) |

### Example 6

1,480 parts of tris(trimethylsiloxy)silylpropylamine represented by the following formula: 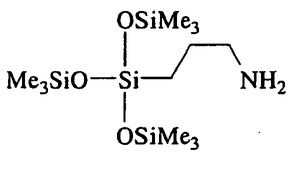 706 parts of octamethylcyclotetrasiloxane and 2.0 parts of 3% potassium siliconate were charged into a separable flask and agitated at 150°C for 5 hours. 3.0 parts of ethylene chlorohydrin was added to the resulting reaction mixture and agitated at 100°C for 1 hour for neutralization. Subsequently, unreacted silane and siloxane were distilled off under reduced pressure to obtain an organopolysiloxane represented by the following average compositional formula:

[(CH₃)₃SiO_{1/2}]₃[(CH₃)₂SiO]₁₀[(CH₂CH₂CH₂NH₂)SiO_{3/2}]₁ .

This product was a colorless transparent liquid and had an amine equivalent of 1,020 g/mol.

300 parts of the thus obtained organopolysiloxane and 38 parts of itaconic acid were charged into a separable flask, followed by agitation at 140°C for 3 hours and distilling off a theoretical amount of water by means of an ester adapter. The thus obtained pyrrolidonecarboxyl group-containing organopolysiloxane was a light yellow transparent liquid having a kinetic viscosity of 270 mm²/s (25°C) wherein CH₂CH₂CH₂NH₂ in the above formula was replaced by 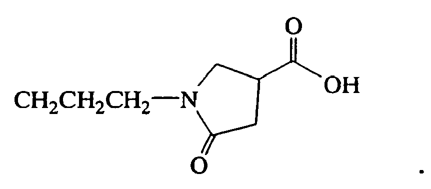 The carboxy equivalent was at 1,300 g/mol. The results of measurement of GPC revealed that the weight average molecular weight was at 1,200. The ¹³C-NMR spectra of the organopolysiloxane are shown in Table 6 below.

**Table 6**

| ¹³C-NMR (CDCl₃) | |
|---|---|
| 1.5 ppm | (Si-CH₃) |
| 12.2 ppm | (Si-CH₂-CH₂-CH₂) |
| 18.9 ppm | (Si-CH₂-CH₂-CH₂) |
| 46.7 ppm | (Si-CH₂-CH₂-CH₂) |
| 172.6 ppm | (N-CO) |
| 36.1 ppm | (CO-CH₂) |
| 45.4 ppm | (N-CH₂) |
| 34.5 ppm | (CH) |
| 178.3 ppm | (COOH) |

### Example 7

34 parts of tris(trimethylsiloxy)silylpropylamine, 422 parts of octamethylcyclotetrasiloxane and 0.8 parts of 3% potassium siliconate were charged into a separable flask and agitated at 150°C for 5 hours. 1.5 parts of ethylene chlorohydrin was added to the resulting reaction mixture and agitated at 100°C for 1 hour for neutralization. Subsequently, unreacted silane and siloxane were distilled off under reduced pressure to obtain an organopolysiloxane represented by the following average compositional formula:

[(CH₃)₃SiO_{1/2}]₃[(CH₃)₂SiO]₆₀[(CH₂CH₂CH₂NH₂)SiO_{3/2}]₁ .

This product was a colorless transparent liquid and had an amine equivalent of 4,540 g/mol.

200 parts of the thus obtained organopolysiloxane and 5.8 parts of itaconic acid were charged into a separable flask, followed by agitation at 140°C for 3 hours and distilling off a theoretical amount of water by means of an ester adapter. The thus obtained pyrrolidonecarboxyl group-containing organopolysiloxane was a light yellow transparent liquid having a kinetic viscosity of 420 mm²/s (25°C) wherein CH₂CH₂CH₂NH₂ in the above formula was replaced by 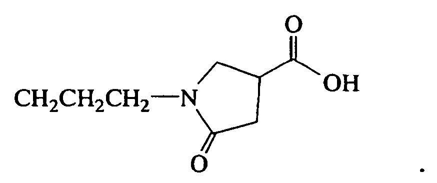 The carboxy equivalent was at 4,670 g/mol. The results of measurement of GPC revealed that the weight average molecular weight was at 4,900. The ¹³C-NMR spectra of the organopolysiloxane are shown below.

**Table 7**

| ¹³C-NMR (CDCl₃) | |
|---|---|
| 1.5 ppm | (Si-CH₃) |
| 12.5 ppm | (Si-CH₂-CH₂-CH₂) |
| 19.2 ppm | (Si-CH₂-CH₂-CH₂) |
| 46.8 ppm | (Si-CH₂-CH₂-CH₂) |
| 172.0 ppm | (N-CO) |
| 36.1 ppm | (CO-CH₂) |
| 45.3 ppm | (N-CH₂) |
| 34.2 ppm | (CH) |
| 178.0 ppm | (COOH) |

### Example 8

16 parts of hexamethyldisiloxane, 304 parts of decamethylcyclooctasiloxane, 36 parts of triethoxysilane [HSi(OC₂H₅)₃], 440 parts of an alkyl-modified organopolysiloxane represented by the following formula:

[(CH₃)₃SiO_{1/2}]₂[(CH₃)₂SiO]₃₁[(CH₃)(C₁₃H₂₇)SiO]₈ .

and 20 parts of concentrated sulfuric acid were charged into a separable flask, in which 8 parts of water was dropped under agitation. After agitating at 25°C for 12 hours, the resulting reaction mixture was washed with water several times, thereby confirming that the mixture became neutral. Subsequently, unreacted silane and siloxanes were distilled off to obtain an organopolysiloxane represented by the following average compositional formula:

[(CH₃)₃SiO_{1/2}]₄[(CH₃)₂SiO]₇₁[(CH₃)(C₁₃H₂₇)SiO]₈[HSiO_{3/2}]₂ .

This product was a colorless transparent liquid whose kinetic viscosity was at 260 mm²/s (25°C).

250 parts of the thus obtained organopolysiloxane and 100 parts of toluene were charged into a separable flask, to which 1 part of a toluene solution of chloroplatinic acid complex (0.5% of platinum) was added. While agitating the mixture at 110°C, 14.2 parts of N-allyl-4-carbomethoxypyrrolidone represented by the following formula: 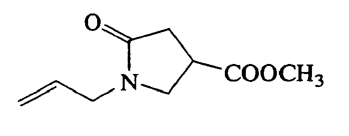 was gently dropped therein.

After agitation at 110°C for 5 hours, unreacted matters were distilled off under reduced pressure to obtain a light yellow transparent liquid. The resulting organopolysiloxane was such that the HSiO_{3/2} unit was replaced by 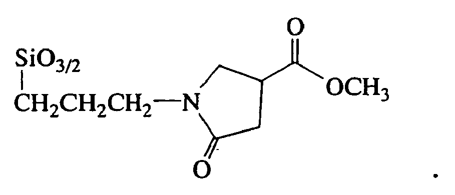 The results of measurement of GPVC revealed that the weight average molecular weight was at 8,000. Moreover, ¹³C-NMR spectra of the organopolysiloxane are shown in Table 8 below.

**Table 8**

| ¹³C-NMR (CDCl₃) | |
|---|---|
| 1. 5 ppm | (Si-CH₃) |
| 11.4 ppm | (Si-CH₂-CH₂-CH₂) |
| 18.5 ppm | (Si-CH₂-CH₂-CH₂) |
| 45.3 ppm | (Si-CH₂-CH₂-CH₂) |
| 172.0 ppm | (N-CO) |
| 35.9 ppm | (CO-CH₂) |
| 45.2 ppm | (N-CH₂) |
| 34.1 ppm | (CH) |
| 178.0 ppm | (COOCH₃) |
| 52.0 ppm | (COOCH₃) |
| 12.4-31.2 ppm | (Si-C₁₃H₂₇) |

### Example 9

40 parts of hexamethyldisiloxane, 1,408 parts of octamethylcyclotetrasiloxane, 38 parts of tetrakis(trimethylsiloxy) silane [HSi(OC₂H₅)₃], 89 parts of triethoxysilane and 20 parts of concentrated sulfuric acid were charged into a separable flask, in which 15 parts of water was dropped under agitation. After agitating at 25°C for 12 hours, the resulting mixture was washed with water several times, thereby confirming that it became neutral. Subsequently, unreacted silanes and siloxanes were distilled off to obtain an organopolysiloxane represented by the following average compositional formula:

[(CH₃)₃SiO_{1/2}]₉[(CH₃)₂SiO]₂₀₀ [HSiO_{3/2}]₅(SiO₂)₁ .

This product was a colorless transparent liquid whose kinetic viscosity was at 540 mm²/s (25°C).

400 parts of the thus obtained organopolysiloxane and 100 parts of toluene were charged into a separable flask, to which 2.0 parts of a toluene solution of chloroplatinic acid complex (0.5% of platinum) was added. While agitating the mixture at 110°C, 26 parts of N-allyl-4-carbomethoxypyrrolidone represented by the following formula: 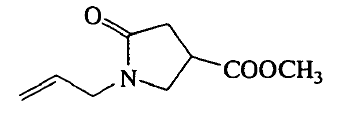 was gently dropped therein.

After agitation at 110°C for 5 hours, unreacted matters were distilled off under reduced pressure to obtain a light yellow transparent liquid. From the results of measurement of GPC, it was confirmed that the weight average molecular weight was at 1,700. Moreover, ¹³C-NMR spectra of the organopolysiloxane are shown in Table 9 below.

**Table 9**

| ¹³C-NMR (CDCl₃) | |
|---|---|
| 1.5 ppm | (Si-CH₃) |
| 12.2 ppm | (Si-CH₂-CH₂-CH₂) |
| 19.0 ppm | (Si-CH₂-CH₂-CH₂) |
| 45.9 ppm | (Si-CH₂-CH₂-CH₂) |
| 171.8 ppm | (N-CO) |
| 36.3 ppm | (CO-CH₂) |
| 45.5 ppm | (N-CH₂) |
| 34.9 ppm | (CH) |
| 178.3 ppm | (COOCH₃) |
| 53.0 ppm | (COOCH₃) |

### Example 10

35 parts of tris(trimethylsiloxy)silylpropylamine, 22 parts of octamethylcyclotetrasiloxane, 45 parts of methyl(aminoethylaminopropyl)polysiloxane represented by the following formula: 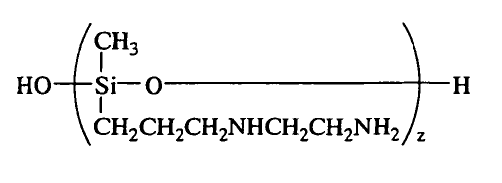 wherein z is an integer, and 0.3 parts of 3% potassium siliconate were charged into a separable flask and agitated at 150°C for 5 hours. 3.0 parts of ethylene chlorohydrin was added to the resulting reaction mixture and agitated at 100°C for 1 hour for neutralization. Subsequently, unreacted silane and siloxane were distilled off under reduced pressure to obtain an organopolysiloxane represented by the following average compositional formula:

[(CH₃)₃SiO_{1/2}]₃[(CH₃)₂SiO]₃[(CH₃)(CH₂CH₂CH₂NHCH₂CH₂NH₂)SiO]₃-[(CH₂CH₂CH₂NH₂)SiO_{3/2}]₁ .

This product was a light yellow transparent liquid and had an amine equivalent of 137 g/mol.

100 parts of the thus obtained organopolysiloxane and 110 parts of dimethyl itaconate were charged, followed by agitating at 110°C for 2 hours and distilling off a theoretical amount of water by means of an ester adapter. The resulting carbomethoxypyrrolidone group-containing organopolysiloxane was such that, in the above formula, (CH₃)(CH₂CH₂CH₂NHCH₂CH₂NH₂)SiO was replaced by 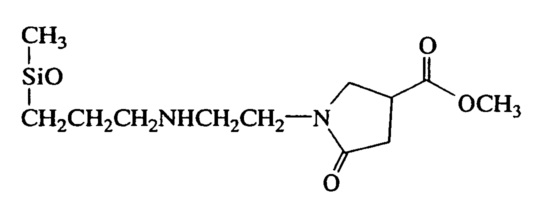 and (CH₂CH₂CH₂NH₂)SiO_{3/2} was replaced by 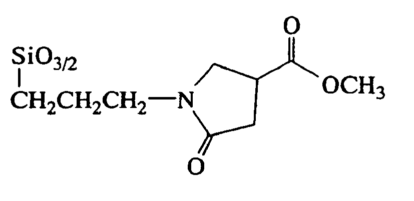 This organopolysiloxane was a light yellow transparent liquid whose kinetic viscosity was at 470 mm²/s (25°C). Form the results of measurement of GPC, it was confirmed that the weight average molecular weight was at 1,600. Moreover, ¹³C-NMR spectra of the organopolysiloxane are shown in Table 10 below.

**Table 10**

| ¹³C-NMR (CDCl₃) | |
|---|---|
| 1.5 ppm | (Si-CH₃) |
| 13.5 ppm | (Si-CH₂-CH₂-CH₂-NCO-) |
| 19.9 ppm | (Si-CH₂-CH₂-CH₂-NCO-) |
| 47.8 ppm | (Si-CH₂-CH₂-CH₂-NCO-) |
| 13.9 ppm | (Si-CH₂-CH₂-CH₂-NH-CH₂-CH₂-) |
| 20.1 ppm | (Si-CH₂-CH₂-CH₂-NH-CH₂-CH₂-) |
| 52.5 ppm | (Si-CH₂-CH₂-CH₂-NH-CH₂-CH₂-) |
| 48.8 ppm | (Si-CH₂-CH₂-CH₂-NH-CH₂-CH₂-) |
| 48.1 ppm | (Si-CH₂-CH₂-CH₂-NH-CH₂-CH₂-) |
| 172.9 ppm | (N-CO) |
| 36.5 ppm | (CO-CH₂) |
| 45.2 ppm | (N-CH₂) |
| 34.1 ppm | (CH) |
| 179.6 ppm | (COOCH₃) |
| 52.1 ppm | (COOCH₃) |

### Comparative Preparatory Example 1

38 parts of itaconic acid was added to 300 parts of an organopolysiloxane represented by the average compositional formula of [(CH₃)₃SiO_{1/2}]₂[(CH₃)₂SiO]₁₀[(CH₃)(CH₂CH₂CH₂NH₂)SiO]₁ and having an amine equivalent of 1,054 g/mol and charged into a separable flask, followed by agitating at 140°C for 3 hours to distill off a theoretical amount water by means of an ester adapter. The resulting linear pyrrolidonecarboxyl group-containing organopolysiloxane was a light yellow transparent liquid whose kinetic viscosity was at 2,800 mm²/s (25°C) and had a carboxy equivalent of 1,220 g/mol.

### Comparative Preparatory Example 2

5.5 parts of itaconic acid was added to 200 parts of an organopolysiloxane represented by the average compositional formula of [(CH₃)₃SiO_{1/2}]₂[(CH₃)₂SiO]₆₀[(CH₃)(CH₂CH₂CH₂NH₂)SiO]₁ and having an amine equivalent of 4,713 g/mol and charged into a separable flask, followed by agitating at 140°C for 3 hours to distill off a theoretical amount water by means of an ester adapter. The resulting linear pyrrolidonecarboxyl group-containing organopolysiloxane was a light yellow transparent liquid whose kinetic viscosity was at 1,800 mm²/s (25°C) and had a carboxy equivalent of 4,840 g/mol.

### Example 11

10 g of the pyrrolidonecarboxyl group-containing organopolysiloxane of Example 7 was dissolved in 50 g of decamethylcyclopentasiloxane, to which 40 g of titanium oxide (MT-100TV, made by Tayca Corporation) was added, followed by dispersion by use of a beads mill to obtain a titanium oxide dispersion (a).

### Example 12

8 g of the pyrrolidonecarboxyl group-containing organopolysiloxane of Example 6 was dissolved in 42 g of decamethylcyclopentasiloxane, to which 50 g of zinc oxide (MZ505S, made by Tayca Corporation) was added, followed by dispersion by use of a beads mill to obtain a zinc oxide dispersion (b).

### Comparative Example 1

10 g of the linear pyrrolidonecarboxyl group-containing organopolysiloxane of Comparative Preparatory Example 2 was dissolved in 50 g of decamethylcyclopentasiloxane, to which 40 g of titanium oxide (MT-100TV, made by Tayca Corporation) was added, followed by dispersion by use of a beads mill to obtain a titanium oxide dispersion (c).

### Comparative Example 2

8 g of the linear pyrrolidonecarboxyl group-containing organopolysiloxane of Comparative Preparatory Example 1 was dissolved in 42 g of decamethylcyclopentasiloxane, to which 50 g of zinc oxide (MZ505S, made by Tayca Corporation) was added, followed by dispersion by use of a beads mill to obtain a zinc oxide dispersion (d).

### Comparative Example 3

10 g of a polyether-modified silicone represented by the following average compositional formula was dissolved in 50 g of decamethylcyclopentasiloxane, to which 40 g of titanium oxide (MT-100TV, made by Tayca Corporation) was added, followed by dispersion by use of a beads mill to obtain a titanium oxide dispersion (e):

[(CH₃)₃SiO_{1/2}]₂[(CH₃)₂SiO]₄₅[(CH₃)(PE)SiO]₃

wherein PE = CH₂CH₂CH₂O(CH₂CH₂O)₉H .

### Evaluation of dispersion:

The powder dispersions and powder compositions of Examples 11, 12 and Comparative Examples 1 to 3 were each mixed with decamethylcyclopentasiloxane so that the powder concentration was made at 5%, and the mixture was placed in a 50 ml sedimentation tube. After 2 days, a sedimentation height at a portion which became opaque by powder sedimentation was visually observed and indicated as a ratio to the total volume. The results are shown in the following table.

**Table 11**

| | Sedimentation Property |
|---|---|
| Example 11 | 0.6 % |
| Example 12 | 1.8 % |
| Comparative Example 1 | 6.7 % |
| Comparative Example 2 | 8.4 % |
| Comparative Example 3 | 9.5 % |

Although the dispersions of Examples 11, 12 were uniform, the sedimentation of the powder was found in Comparative Examples 1 to 3 and thus, the dispersions were non-uniform.

### Examples 13, 14 and Comparative Examples 4 to 6

Sunscreen agents having the formulations indicated in Table 12 were prepared and the qualities thereof were evaluated. It will be noted that the unit is "parts."

**Table 12**

| | | | Example | | Comparative Example | | |
|---|---|---|---|---|---|---|---|
| | | | 13 | 14 | 4 | 5 | 6 |
| 1 | KF96 6cs (Note 1) | | 5.0 | 5.0 | 5.0 | 5.0 | 5.0 |
| 2 | KSG-210 (Note 2) | | 5.0 | 5.0 | 5.0 | 5.0 | 5.0 |
| 3 | Glyceryl triisooctanoate | | 3.0 | 3.0 | 3.0 | 3.0 | 3.0 |
| 4 | KF-6019 (Note 3) | | 1.0 | 1.0 | 1.0 | 1.0 | 1.0 |
| 5 | Octyl para-methoxycinnamate | | 6.0 | 6.0 | 6.0 | 6.0 | 6.0 |
| 6 | Sodium chloride | | 0.5 | 0.5 | 0.5 | 0.5 | 0.5 |
| 7 | 1,3-Butylene glycol | | 2.0 | 2.0 | 2.0 | 2.0 | 2.0 |
| 8 | Purified water | | Balance | Balance | Balance | Balance | Balance |
| 9 | Perfume | | Appropriate amount | Appropriate amount | Appropriate amount | Appropriate amount | Appropriate amount |
| 10 | Titanium oxide dispersion (a) [Example 11] | | 50 | | | | |
| 11 | Zinc oxide dispersion (b) [Example 12] | | | 50 | | | |
| 12 | Titanium oxide dispersion (c) [Comparative Example 1] | | | | 50 | | |
| 13 | Zinc oxide dispersion (d) [Comparative Example 2] | | | | | 50 | |
| 14 | Titanium oxide dispersion (e) [Comparative Example 3] | | | | | | 50 |

| | Results of evaluation | | | | | | |
|---|---|---|---|---|---|---|---|
| 1 | Dispersion stability | | ⊚ | ⊚ | △ | △ | △ |
| 2 | Use feelings | Dry feel | ⊚ | ⊚ | △ | △ | △ |
| | | Spreading | ○ | ⊚ | ○ | ○ | ○ |
| | | Transparency of cosmetic film | ⊚ | ○ | ○ | ○ | × |
| | | No greasiness | ⊚ | ⊚ | △ | △ | × |
| | | Sunscreen effect | ⊚ | ⊚ | ○ | ○ | ○ |
| | | Makeup lasting performance | ⊚ | ⊚ | △ | △ | △ |

| | | | | | | | |
|---|---|---|---|---|---|---|---|
| (Note 1) KF96: dimethylpolysiloxane (made by Shin-Etsu Chemical Co., Ltd.) (Note 2) KSG-210: dimethicone (PEG-10-15) crosspolymer (Shin-Etsu Chemical Co., Ltd.) (Note 3) KF-6019: PEG-10 dimethicone (made by Shin-Etsu Chemical Co., Ltd.) | | | | | | | |

### (Preparation method)

A: 1, 2, 3 and 4 were mixed uniformly.
B: 6, 7 and 8 were mixed uniformly.
C: B was added to A and emulsified.
D: 5, 9 and 10 to 14 were added to C to obtain a sunscreen agent.

The qualities indicated in Table 12 were evaluated in the following way.

### 1. Dispersion stability of powder

Each sunscreen agent was allowed to stand at room temperature for one month, followed by observation of the degree of coagulation of the powder and judgment based on the following standards.

### Evaluation standards

⊚: Powder coagulation was not observed.
○: Coagulation was slightly observed.
△: A tendency toward powder coagulation was observed.
×: Powder coagulation was appreciably observed.

### 2. Standards for use feeling

Each sunscreen agent was subjected to five-grade evaluation by 50 female panelists based on the following standards with respect to dry feel, spreading, cosmetic film transparency, skin greasiness, sunscreen effect and makeup lasting performance. The resulting point average was judged in terms of ○, ×, etc., according to the following standards in every example.

### Evaluation standards

5 points: Good
4 points: Slightly good
3 points: Moderate
2 points: Slightly poor
1 point: Poor

### Judgment of the point average

⊚: The point average obtained is not smaller than 4.5.
○: The point average is from not smaller than 3.5 to smaller than 4.5.
△: The point average is from not smaller than 2.5 to smaller than 3.5.
×: The point average is from not smaller than 1.5 to smaller than 2.5.

As will be apparent from the results of Table 12, the powders of Examples 13, 14 of the invention were free of coagulation and excellent in dispersability. The use feelings were good in respect of all the entries.

### Example 15 and Comparative Example 7

Milky lotions having the formulations of Table 13 were prepared to evaluate qualities thereof. It will be noted that the unit is "parts."

**Table 13**

| Ingredients | | | Example 15 | Comparative Example 7 |
|---|---|---|---|---|
| 1 | Decamethylcyclopentasiloxane | | 15.0 | 15.0 |
| 2 | Methylphenylpolysiloxane | | 5.0 | 5.0 |
| 3 | Squalene | | 5.0 | 5.0 |
| 4 | Pentaerythritol tetra-2-ethylhexanoate | | 5.0 | 5.0 |
| 5 | Pyrrolidonecarboxyl group-containing organopolysiloxane of Example 6 | | 3.0 | - |
| 6 | Pyrrolidonecarboxyl group-containing organopolysiloxane of Comparative Preparatory Example 1 | | - | 3.0 |
| 7 | Spherical powder of organopolysiloxane elastomer (Note 1) | | 2.0 | 2.0 |
| 8 | Hydrophobicized silica (Note 2) | | 0.5 | 0.5 |
| 9 | Magnesium ascorbate phosphate | | 1.0 | 1.0 |
| 10 | Sodium chloride | | 1.0 | 1.0 |
| 11 | Polyethylene glycol 11000 | | 1.0 | 1.0 |
| 12 | Propylene glycol | | 8.0 | 8.0 |
| 13 | Preservative | | Appropriate amount | Appropriate amount |
| 14 | Perfume | | Appropriate amount | Appropriate amount |
| 15 | Purified water | | 53.5 | 53.5 |

| | Results of evaluation | | | |
|---|---|---|---|---|
| 1 | Emulsified state | | ⊚ | △ |
| 2 Use Feelings | | Dry feel | ⊚ | △ |
| | | Spreading | ⊚ | ○ |
| | | No greasiness | ⊚ | △ |
| | | Makeup lasting performance | ⊚ | ○ |

| | | | | |
|---|---|---|---|---|
| (Note 1) Spherical powder of organopolysiloxane elastomer: KMP590 (Shin-Etsu Chemical Co., Ltd.) (Note 2) Hydrophobicized silica: Aerosil R972 (made by Japan Aerosil Co., Ltd.) | | | | |

### (Preparation method)

A: Ingredients 1 to 5 and 6 were mixed uniformly, to which ingredients 7, 8 were uniformly added and dispersed.
B: Ingredients 9 to 11 were added to and dissolved in ingredient 15, to which ingredients 11, 12 were added after uniform mixing thereof.
C: B was gradually added to A and emulsified, followed by cooling and further adding ingredient 14 to obtain a milky lotion.

The qualities in Table 13 were evaluated in the following way.

### 1. Emulsified state

Each lotion was allowed to stand at room temperature for 1 month and observed with respect to the state of separation, followed by judging according to the following standards.

### Evaluation standards

⊚: Good
○: Barely separated
△: Slightly separated
×: Completely separated

### 2. Evaluation of Use feelings

The milky lotions obtained were each subjected to five-grade evaluation by 50 female panelists based on the following standards with respect to dry feel, spreading, skin greasiness and makeup lasting performance. The resulting point average was judged in terms of ○, ×, etc., according to the following standards in every example.

### Evaluation standards:

5 points: Good
4 points: Slightly good
3 points: Moderate
2 points: Slightly poor
1 point: Poor

### Judgment of the point average

⊚: The point average obtained is not smaller than 4.5.
○: The point average is from not smaller than 3.5 to smaller than 4.5.
△: The point average is from not smaller than 2.5 to smaller than 3.5.
×: The point average is from not smaller than 1.5 to smaller than 2.5.

As will be apparent from Table 13, the lotion of Example 5 is more stabilized in emulsification than the lotion of Comparative Example 7 and has been confirmed to be more excellent in the use feelings as well.

### Example 16 and Comparative Example 8

Hair creams indicated in Table 14 were prepared and the qualities thereof were evaluated. It will be noted that the unit is "parts."

**Table 14**

| Ingredients | | | Example 16 | Comparative Example 8 |
|---|---|---|---|---|
| 1 | Decamethylcyclopentasiloxane | | 10.0 | 10.0 |
| 2 | Methylphenylpolysiloxane | | 5.0 | 5.0 |
| 3 | Squalane | | 4.0 | 4.0 |
| 4 | Silicone network resin (Note 1) | | 1.0 | 1.0 |
| 5 | Glyceryl dioleate | | 2.0 | 2.0 |
| 6 | Organopolysiloxane of Example 8 | | 4.0 | - |
| 7 | Organopolysiloxane of Comparative Preparatory Example 2 | | - | 4.0 |
| 8 | Sodium sorbitol sulfate | | 2.0 | 2.0 |
| 9 | Sodium condroitin sulfate | | 1.0 | 1.0 |
| 10 | Sodium hyalurate | | 0.5 | 0.5 |
| 11 | Propylene glycol | | 3.0 | 3.0 |
| 12 | Preservative | | 1.5 | 1.5 |
| 13 | Vitamin E acetate | | 0.1 | 0.1 |
| | 14 Antioxidant | | Appropriate amount | Appropriate amount |
| 15 | Perfume | | Appropriate amount | Appropriate amount |
| 16 | Purified water | | 65.9 | 65.9 |

| Results of evaluation | | | | |
|---|---|---|---|---|
| Use Feelings | | Spreading upon application | ⊚ | △ |
| | | Flexibility after use | ⊚ | △ |
| | | Hair hold | ⊚ | ○ |
| | | Moistness | ⊚ | ○ |
| | | Finger combing | ⊚ | ○ |
| | | Smoothness lasting | ⊚ | △ |

| | | | | |
|---|---|---|---|---|
| (Note 1) Silicone network resin: 50%-D5 solution of a silicone network resin having a [Me₃SiO_{1/2}]/[SiO₂] value of 0.8 | | | | |

### (Preparation method)

A: Ingredients 1 to 6 and 7 and ingredients 12 to 14 were mixed under heating conditions.
B: Ingredients 8 to 11 and 16 were mixed under heating conditions.
C: B was gradually added to A under agitation and emulsified, followed by cooling and further addition of ingredient 15 to obtain a hair cream.

### 1. Evaluation of use feelings

The resulting hair creams were each subjected to five-grade evaluation by 50 female panelists according to the following standards with respect to spreading upon application, flexibility after use, hair hold, moistness, finger combing and smoothness lasting. The resulting point average was judged in terms of ○, ×, etc., according to the following standards in every example.

### Evaluation standards

5 points: Good
4 points: Slightly good
3 points: Moderate
2 points: Slightly poor
1 point: Poor

### Judgment of the point average

⊚: The point average obtained is not smaller than 4.5.
○: The point average is from not smaller than 3.5 to smaller than 4.5.
△: The point average is from not smaller than 2.5 to smaller than 3.5.
×: The point average is from not smaller than 1.5 to smaller than 2.5.

As will be apparent from Table 14, it has been confirmed that the hair cream obtained in Example 16 is much more excellent than that of Comparative Example 8 with respect to the smoothness lasting and the like in the evaluation of the use feelings made by the panelists.

### Example 17

Makeup remover

| (Ingredients) | | (%) |
|---|---|---|
| 1. | POE (10) sorbitan monolaurate | 10.0 |
| 2. | Pyrrolidonecarboxyl group-containing organopolysiloxane of Example 6 | 20.0 |
| 3. | Sorbitol | 10.0 |
| 4. | Carageenan | 0.5 |
| 5. | Glycerine | 5.0 |
| 6. | Sodium citrate | 0.5 |
| 7. | Preservative | appropriate amount |
| 8. | Perfume | appropriate amount |
| 9. | Purified water | 54.0 |

### (Preparation method)

A: Ingredients 1 to 7 and 9 were mixed together and dissolved uniformly.
B:Ingredient 8 was added to A to obtained a makeup remover.

The thus obtained makeup remover was used to remove a foundation, whereupon this makeup remover was found to well fit with the foundation and allow the foundation to be well taken off, to be diffusively spread upon use and become moistened after the removal and thus to be very good at usability and use feeling.

### Example 18

Hair style remover

| (Ingredients) | | (%) |
|---|---|---|
| 1. | Polyoxyethylene (15) isocetyl ether (Note 1) | 10.0 |
| 2. | Pyrrolidonecarboxyl group-containing organopolysiloxane of Example 7 | 20.0 |
| 3. | 1,3-Butylene glycol | 10.0 |
| 4. | Glycerine | 10.0 |
| 5. | Carageenan | 0.5 |
| 6. | Sodium chloride | 0.5 |
| 7. | Preservative | appropriate amount |
| 8. | Perfume | appropriate amount |
| 9. | Purified water | 49.0 |

| | | |
|---|---|---|
| (Note 1) Polyoxyethylene (15) isocetyl ether: made by Sanyo Chemical Industries, Ltd. | | |

### (Preparation method)

A: Ingredients 1 to 7 and 9 were added and uniformly dissolved.
B: Ingredient 8 was added to A to obtain a hair style remover.

The thus obtained hair style remover was used to wash the hair, revealing that the hair style remover ensured good hair styling, were well fit with the skin oils, and allowed the skin oils to be taken off well. In addition, the remover was diffusively spread upon use and was free of greasiness after use and could keep the washed skin moist, thus being very good at usability and use feeling.

### Example 19

Facial wash

| (Ingredients) | | (%) |
|---|---|---|
| 1. | Polyoxyethylene (6) lauryl ether (Note 1) | 5.0 |
| 2. | Organopolysiloxane of Example 9 | 10.0 |
| 3. | Ethanol | 10.0 |
| 4. | Lauryl dimethylamine oxide (Note 2) | 2.0 |
| 5. | Propylene glycol | 3.0 |
| 6. | Sodium citrate | 0.5 |
| 7. | Preservative | appropriate amount |
| 8. | Perfume | appropriate amount |
| 9. | Purified water | 69.5 |

| | | |
|---|---|---|
| (Note 1) Polyoxyethylene (6) lauryl ether: Pegnol L-6 (made by Toho Chemical Industry Co., Ltd.) (Note 2) Lauryl dimethylamine oxide: Unisafe A-LM (made by NOF Corporation) | | |

### (Preparation method)

A: Ingredients 1 to 7 and 9 were added and dissolved uniformly.
B: Ingredient 8 was added to A to obtain a facial wash.

When using the thus obtained facial wash, it was found that the facial wash was well fit with other types of cosmetics and the skin oils and allowed the dirt to be taken off very well. In addition, the thus obtained facial wash was diffusively spread upon use and was free of greasiness after use and could keep the applied skin moist with no greasiness, thus being very good at usability and use feeling.

### Example 20

Makeup remover

| (Ingredients) | | (%) |
|---|---|---|
| 1. | Polyoxyethylene (6) sorbitan monolaurate (Note 1) | 5.0 |
| 2. | Pyrrolidonecarboxyl group-containing organopolysiloxane of Example 6 | 5.0 |
| 3. | Organopolysiloxane of Example 10 | 5.0 |
| 4. | Ethanol | 10.0 |
| 5. | Glycerine | 2.0 |
| 6. | Dipropylene glycol | 3.0 |
| 7. | Sodium glutamate | 0.5 |
| 8. | Preservative | appropriate amount |
| 9. | Perfume | appropriate amount |
| 10. | Purified water | 59.5 |

| | | |
|---|---|---|
| (Note 1) Polyoxyethylene (6) sorbitan monolaurate: (made by Sanyo Chemical Industries, Ltd.) | | |

### (Preparation method)

A: Ingredients 1 to 8 and 10 were added and dissolved uniformly.
B: Ingredient 9 was added to A to obtain a makeup remover.

When using the thus obtained makeup remover, it was found that the thus obtained makeup remover was well fit with other types of cosmetics and the skin oils and allowed the dirt to be taken off very well. In addition, the makeup remover was well spread upon use and was free of greasiness after use and could keep the applied skin moist with no greasiness, thus being very good at usability and use feeling.

### Example 21

Polyhydric alcohol-in-oil emulsified cosmetic

| (Ingredients) | | (%) |
|---|---|---|
| 1. | Crosslinking dimethylpolysiloxane (Note 1) | 30.0 |
| 2. | Decamethylcyclopentanesiloxane | 15.0 |
| 3. | Dimethylsiloxane (6 mm²/s (25°C)) | 7.0 |
| 4. | Pyrrolidonecarboxyl group-containing organopolysiloxane of Example 7 | 3.0 |
| 5. | Dimethyldistearylammonium hectrite | 2.0 |
| 6. | Preservative | appropriate amount |
| 7. | Perfume | appropriate amount |
| 8. | Sodium chloride | 0.05 |
| 9. | 1,3-Butylene glycol | 42.95 |

| | | |
|---|---|---|
| (Note 1) Crosslinking dimethylpolysiloxane: KSG15 (made by Shin-Etsu Chemical Co., Ltd.) | | |

### (Preparation method)

A: Ingredients 1 to 5 and 7 were uniformly mixed.
B: Ingredients 6, 8 and 9 were mixed together.
C: B was added to A and uniformly emulsified.

It was confirmed that the thus obtained polyhydric alcohol-in-oil emulsified cosmetic was diffusively spread and was free of greasiness and oiliness, and could keep the applied skin moist, thus being a very stable polyhydric alcohol-in-nonaqueous oil emulsified cosmetic.

### Example 22

Polyhydric alcohol-in solid oil emulsified rouge

| (Ingredients) | | (%) |
|---|---|---|
| 1. | Crosslinking dimethylpolysiloxane (Note 1) | 5.0 |
| 2. | Decamethylcyclopentasiloxane | 5.0 |
| 3. | Dimethylpolysiloxane (6 mm²/s (25°C)) | 19.7 |
| 4. | Cetyl isooctanoate | 15.0 |
| 5. | Paraffin wax (melting point of 80°C) | 12.0 |
| 6. | Organopolysiloxane of Example 8 | 3.0 |
| 7. | Dimethyldistearylammonium hectrite | 0.2 |
| 8. | Hydrophobicized powder | 25.0 |
| 9. | Sodium citrate | 0.1 |
| 10. | Preservative | appropriate amount |
| 11. | Perfume | appropriate amount |
| 12. | 1,3-Butylene glycol | 15.0 |

| | | |
|---|---|---|
| (Note 1) Crosslinking dimethylpolysiloxane: KSG15 (made by Shin-Etsu Chemical Co., Ltd.) | | |

### (Preparation method)

A: Ingredients 1 to 7 and 11 were heated to 80°C and mixed uniformly.
B: Ingredient 8 was added to A and uniformly dispersed.
C: Ingredients 9, 10 and 12, which had been heated to 80°C beforehand, were added to B and emulsified, followed by pouring into a metal dish and cooled.

It was confirmed that the thus obtained polyhydric alcohol-in-solid oil rouge was diffusively spread and run without greasiness and oiliness and could keep the applied skin moist, thus being a very stable polyhydric alcohol-in-solid oil rouge.

### Example 23

Creamy rouge

| (Ingredients) | | (%) |
|---|---|---|
| 1. | Dextrin palmitate/ethylhexanoate (Note 1) | 9.0 |
| 2. | Glyceryl triisooctanoate | 22.0 |
| 3. | Bentonite | 0.7 |
| 4. | Organopolysiloxane of Example 8 | 1.5 |
| 5. | Decamethylcyclopentasiloxane | 42.0 |
| 6. | 1,3-Butylene glycol | 5.0 |
| 7. | Sodium chloride | 0.5 |
| 8. | Purified water | 19.3 |
| 9. | Colored pigment | appropriate amount |

| | | |
|---|---|---|
| (Note 1) Dextrin palmitate/ethylhexanoate: Leopal TT (made by Chiba Flour Milling Co., Ltd.) | | |

### (Preparation method)

A: Ingredient 1, part of ingredient 2, and ingredients 3 to 5 were mixed and dissolved.
B: Ingredient 9 was mixed with the balance of ingredient 2 and dispersed by means of a roller.
C: B was added to A and uniformly mixed.
D: Ingredients 6 to 8 were mixed together and heated.
E: D was added to C and emulsified.

It was confirmed that the thus obtained rouge was a W/O type of creamy rouge that was excellent in cosmetic persistency, and was diffusively spread and run without greasiness and oiliness.

### Example 24

Eye liner

| (Ingredients) | | (%) |
|---|---|---|
| 1. | Octamethylcyclotetrasiloxane | 53.5 |
| 2. | Pyrrolidonecarboxyl group-containing organopolysiloxane of Example 7 | 3.0 |
| 3. | Silicone network resin (Note 1) | 15.0 |
| 4. | Dimethyldistearylammonium hectrite | 3.0 |
| 5. | Silicone-treated black iron oxide (Note 2) | 10.0 |
| 6. | 1,3-Butylene glycol | 5.0 |
| 7. | Sodium sulfate | 0.5 |
| 8. | Preservative | appropriate amount |
| 9. | Purified water | 10.0 |

| | | |
|---|---|---|
| (Note 1) Silicone network resin: 50%-D5 solution of a silicone network resin whose value of [Me₃SiO_{1/2}]/[SiO₂] was 0.8. (Note 2) Silicone-treated black iron oxide: one obtained by adding methyl hydrogen polysiloxane in an amount of 2% relative to black iron oxide and subjecting to heat treatment. | | |

### (Preparation method)

A: Ingredients 1 to 4 were mixed, to which ingredient 5 was added, followed by uniform mixing and dispersion.
B: Ingredients 6 to 9 were mixed together.
C: B was gently added to A and emulsified to obtain an eye liner.

It was confirmed that the thus obtained eye liner was diffusively spread and was easy for drawing, had a cool sensation and felt dry with a greasiness-free use feeling, and showed no temperature or aging change with usability and stability being both very excellent. The eye line was also excellent in water proofing and sweat resistance and ensured a very good makeup lasting performance.

### Example 25

Eye shadow

| (Ingredients) | | (%) |
|---|---|---|
| 1. | Decamethylcyclopentasiloxane | 15.0 |
| 2. | Dimethylpolysiloxane (6 mm²/s (25°C)) | 10.0 |
| 3. | Organopolysiloxane of Example 8 | 2.0 |
| 4. | PEG (10) lauryl ether | 0.5 |
| 5. | Silicone-treated chromium oxide (Note 1) | 6.2 |
| 6. | Silicone-treated ultramarine blue (Note 1) | 4.0 |
| 7. | Silicone-treated titanium-coated mica (Note 1) | 6.0 |
| 8. | Sodium chloride | 2.0 |
| 9. | Propylene glycol | 8.0 |
| 10. | Preservative | appropriate amount |
| 11. | Perfume | appropriate amount |
| 12. | Purified water | 46.3 |

| | | |
|---|---|---|
| (Note 1) Silicone treatment: Methyl hydrogen polysiloxane was added in an amount of 3% relative to the powder, followed by heat treatment. | | |

### (Preparation method)

A: Ingredients 1 to 4 were mixed together, to which ingredients 5 to 7 were added and dispersed uniformly.
B: Ingredients 8 to 10 and 12 were dissolved uniformly.
C: Under agitation, B was gently added to A and emulsified, to which ingredient 11 was added thereby obtaining an eye shadow.

It was confirmed that the thus obtained eye shadow was diffusively spread, was free of oiliness and ashiness and ensured fresh and dry use feelings. Additionally, the eye shadow was felt moist, was good at water proofing, water repellency and sweat resistance, was unlikely to suffer coming-off, showed no temperature or aging change and was thus good at stability.

### Example 26

Suncut cream

| (Ingredients) | | (%) |
|---|---|---|
| 1. | Decamethylcyclopentahexane | 17.5 |
| 2. | Acrylic silicone resin/decamethylcyclopenta-siloxane (Note 1) | 12.0 |
| 3. | Glyceryl triisooctanoate | 5.0 |
| 4. | Octyl p-methoxycinnamate | 6.0 |
| 5. | Crosslinking polyether-modified silicone (Note 2) | 5.0 |
| 6. | Pyrrolidonecarboxyl group-containing organopolysiloxane of Example 6 | 1.0 |
| 7. | Oleophilicized zinc oxide | 20.0 |
| 8. | Sodium chloride | 0.5 |
| 9. | 1,3-Butylene glycol | 2.0 |
| 10. | Preservative | appropriate amount |
| 11. | Perfume | appropriate amount |
| 12. | Purified water | 31.0 |

| | | |
|---|---|---|
| (Note 1) Acrylic Silicone resin/decamethylcyclopentasiloxane: KP545 (made by Shin-Etsu Chemical Co., Ltd.) (Note 2) Crosslinking polyether-modified silicone: KSG21 (made by Shin-Etsu Chemical Co., Ltd.) | | |

### (Preparation method)

A: Ingredient 2 was added to part of ingredient 1, to which ingredient 7 was added and dispersed by means of a beads moll.
B: The balance of ingredient 1 and ingredients 3 to 6 were uniformly mixed together.
C: Ingredients 8 to 10 and 12 were mixed together and dissolved uniformly.
D: C was added to B and emulsified, followed by further addition of A and ingredient 11 to obtain a suncut cream.

It was confirmed that the thus obtained cream was free of greasiness and was diffusively spread and run with good adherability and that the cream was well fit, ensured a glossy finish, was very excellent in makeup lasting performance and was very stable with respect to temperature and aging.

### Example 27

Suntan cream

| (Ingredients) | | (%) |
|---|---|---|
| 1. | Decamethylcyclopentanesiloxane | 15.0 |
| 2. | Dimethylpolysiloxane (6 mm²/s (25°C)) | 5.0 |
| 3. | Stearyl-modified acrylic silicone (Note 1) | 0.5 |
| 4. | Organopolysilicone of Example 10 | 6.0 |
| 5. | Palmitic acid | 0.2 |
| 6. | Dimethyloctyl p-aminobenzoate | 0.5 |
| 7. | 4-t-Butyl-4'-methoxy-dibenzoylmethane | 0.5 |
| 8. | Kaolin | 0.5 |
| 9. | Colcothar | 0.2 |
| 10. | Yellow iron oxide | 0.3 |
| 11. | Black iron oxide | 0.1 |
| 12. | Titanium oxide-coated mica | 1.0 |
| 13. | Sodium L-glutamate | 3.0 |
| 14. | 1,3-Butylene glycol | 5.0 |
| 15. | Dioctadecyldimethylammonium chloride | 0.1 |
| 16. | Antioxidant | appropriate amount |
| 17. | Preservative | appropriate amount |
| 18. | Perfume | appropriate amount |
| 19. | Purified water | 62.1 |

| | | |
|---|---|---|
| (Note 1) Stearyl-modified acrylic silicone: KP561 (made by Shin-Etsu Chemical Co., Ltd.) | | |

### (Preparation method)

A: Ingredients 1 to 7 and 16, 17 were dissolved under heating conditions.
B: Ingredient 15 and part of ingredient 19 were heated under agitation, to which ingredients 8 to 12 were added and dispersed.
C: Ingredients 13, 14 and the balance of ingredient 19 were uniformly dissolved and mixed with B.
D: Under agitation, C was gently added to A and emulsified, followed by cooling and further addition of ingredient 18 to obtain a suntan cream.

It was confirmed that the thus obtained suntan cream was fine in texture, was diffusively spread and run, was free of greasiness and oiliness, and ensured moist, fresh and dry use feelings. Additionally, the cream was well fit, had a good makeup lasting performance and did not undergo temperature and aging changes such as of separation and powder coagulation, thus with excellent stability.

### Example 28

Foundation

| (Ingredients) | | (%) |
|---|---|---|
| 1. | Decamethylcyclopentasiloxane | 45.0 |
| 2. | Dimethylpolysiloxane (6 mm²/s (25°C)) | 5.0 |
| 3. | Pyrrolidonecarboxyl group-containing organopolysiloxane of Example 6 | 1.5 |
| 4. | Pyrrolidonecarboxyl group-containing organopolysiloxane of Example 7 | 0.5 |
| 5. | Dimethyldistearylammonium hectrite | 4.0 |
| 6. | Hydrophobicized titanium oxide (Note 1) | 10.0 |
| 7. | Hydrophobicized talc (Note 1) | 6.0 |
| 8. | Hydrophobicized mica (Note 1) | 6.0 |
| 9. | Hydrophobicized colcothar (Note 1) | 1.6 |
| 10. | Hydrophobicized yellow iron oxide (Note 1) | 0.7 |
| 11. | Hydrophobicized black iron oxide (Note 1) | 0.2 |
| 12. | Dipropylene glycol | 5.0 |
| 13. | Methyl p-oxybenzoic acid ester | 0.3 |
| 14. | 2-Amino-2-methyl-1,3-propanediol | 0.2 |
| 15. | Hydrochloric acid | 0.1 |
| 16. | Perfume | appropriate amount |
| 17. | Water | 13.9 |

| | | |
|---|---|---|
| (Note 1) Hydrophobicization treatment: Methyl hydrogen polysiloxane was added in an amount of 2% relative to powder, followed by heat treatment. | | |

### (Preparation method)

A: Ingredients 1 to 5 were mixed under heating conditions, to which ingredients 6 to 11 were added thereby providing a uniform mixture.
B: Ingredients 12 to 15 and 17 were dissolved under heating conditions (the pH of an aqueous mixture was 9.0).
C: Under agitation, B was gently added to A, followed by cooling and further addition of ingredient 16 to obtain a foundation.

It was confirmed that the thus obtained foundation was fine in texture, was diffusively spread and run, was free of greasiness and oiliness and ensured moist, fresh and dry use feelings. In addition, the foundation showed a good makeup lasting performance, underwent no temperature and aging change and was excellent in stability.

### Example 29

Liquid foundation

| (Ingredients) | | (%) |
|---|---|---|
| 1. | Decamethylcyclopentasiloxane | 16.0 |
| 2. | Dimethylpolysiloxane (6 mm²/s (25°C)) | 8.0 |
| 3. | Octyl p-methoxycinnamate | 3.0 |
| 4. | 12-Hydroxystearic acid | 1.0 |
| 5. | Pyrrolidonecarboxyl group-containing organopolysiloxane of Example 7 | 2.0 |
| 6. | Fluorine-modified silicone (Note 1) | 5.0 |
| 7. | Spherical silicone resin powder (Note 2) | 3.0 |
| 8. | Fluorine compound-treated fine-particle titanium oxide (Note 3) | 8.0 |
| 9. | Fluorine compound-treated fine-particle titanated mica (Note 3) | 1.0 |
| 10. | Fluorine compound-treated titanium oxide (Note 3) | 5.0 |
| 11. | Fluorine compound-treated colcothar (Note 3) | 0.9 |
| 12. | Fluorine compound-treated yellow iron oxide (Note 3) | 2.0 |
| 13. | Fluorine compound-treated black iron oxide (Note 3) | 1.0 |
| 14. | Ethanol | 15.0 |
| 15. | Glycerine | 3.0 |
| 16. | Magnesium sulfate | 1.0 |
| 17. | Preservative | appropriate amount |
| 18. | Perfume | appropriate amount |
| 19. | Purified water | 25.1 |

| | | |
|---|---|---|
| (Note 1) fluorine-modified silicone: FL-5 (made by Shin-Etsu Chemical Co., Ltd.) (Note 2) Spherical silicone resin powder: KMP590 (made by Shin-Etsu Chemical Co., Ltd.) (Note 3) Fluorine compound treatment: coated with a diethanolamine perfluoroalkylethylphosphate by 5%. | | |

### (Preparation method)

A: Ingredients 7 to 13 were uniformly mixed together.
B: Ingredients 1 to 6 were heated to 70°C and mixed, to which A was added, followed by uniform dispersion and mixing.
C: Ingredients 14 to 17 and 19 were heated to 40°C and gently added to B and emulsified, followed by cooling and further addition of ingredient 18 to obtain a liquid foundation.

It was confirmed that the thus obtained liquid foundation was free of greasiness and was diffusively spread and run, and was felt dry and had a high sensation of coolness. The foundation did not undergo temperature and aging changes and was excellent in stability.

### Example 30

Moisture cream

| (Ingredients) | | (%) |
|---|---|---|
| 1. | Decamethylcyclopentasiloxane | 10.0 |
| 2. | Methylphenylpolysiloxane | 3.0 |
| 3. | Liquid paraffin | 5.0 |
| 4. | Stearoxy-modified silicone (Note 1) | 8.0 |
| 5. | Organopolysiloxane of Example 10 | 1.5 |
| 6. | Spherical powder of organopolysiloxane elastomer (Note 2) | 2.5 |
| 7. | Hydrophobicized silica (Note 3) | 2.0 |
| 8. | Zinc stearate | 2.0 |
| 9. | Vitamin E acetate | 3.0 |
| 10. | Polyethylene glycol 400 | 1.0 |
| 11. | Sodium lactate | 1.0 |
| 12. | 1,3-Butylene glycol | 5.0 |
| 13. | Preservative | appropriate amount |
| 14. | Perfume | appropriate amount |
| 15. | Purified water | 56.0 |

| | | |
|---|---|---|
| (Note 1) Stearoxy-modified silicone: KF-7002 (Shin-Etsu Chemical Co., Ltd.) (Note 2) Spherical powder of organopolysiloxane elastomer: KMP-590 (Shin-Etsu Chemical Co., Ltd.) (Note 3) Hydrophobicized silica: Aerosil R972 (made by Nippon Aerosil Co., Ltd.) | | |

### (Preparation method)

A: Ingredients 1 to 5 and ingredients 8, 9 were uniformly mixed, to which ingredients 6, 7 were added and uniformly dispersed.
B: Ingredients 10 to 13 and 15 were added and dissolved.
C: B was gently added to A and cooled down, followed by addition of ingredient 14 to obtain a moisture cream.

It was confirmed that the thus obtained moisture cream was diffusively spread and run, was felt fresh and refreshed, was free of greasiness and moist, and did not undergo temperature and aging changes, thus being very excellent in usability and stability.

### Example 31

Hand cream

| (Ingredients) | | (%) |
|---|---|---|
| 1. | Decamethylcyclopentasiloxane | 30.0 |
| 2. | Liquid paraffin | 10.0 |
| 3. | Amino-modified silicone gum (amine equivalent of 70,000 g/mol) | 15.0 |
| 4. | Pyrrolidonecarboxyl group-containing organopolysiloxane of Example 7 | 4.0 |
| 5. | Distearyldimethylammonium chloride | 0.8 |
| 6. | Vitamin E acetate | 0.1 |
| 7. | Polyethylene glycol 4,000 | 1.0 |
| 8. | Glycerine | 10.0 |
| 9. | Aluminum magnesium silicate | 1.2 |
| 10. | Preservative | appropriate amount |
| 11. | Perfume | appropriate amount |
| 12. | Purified water | 27.9 |

### (Preparation method)

A: Ingredients 1,3 were mixed and dissolved under heating conditions, to which ingredients 2, 4 to 6 and 10 were added under heating conditions.
B: Ingredients 7 to 9 and 12 were mixed under heating conditions.
C: B was gently added to A and emulsified, followed by cooling and further addition of ingredient 11 to obtain a hand cream.

It was confirmed that the thus obtained hand cream was free of greasiness and was diffusively spread and run with a moist use feeling, and that the cream was able to effectively protect the skin from a wet work and was very excellent in temperature stability.

### Example 32

O/W hand cream

| (Ingredients) | | (%) |
|---|---|---|
| 1. | Acrylic silicone resin/decamethylcyclopentasiloxane (Note 1) | 10.0 |
| 2. | Stearyl-modified acrylic silicone resin (Note 2) | 8.0 |
| 3. | Cetanol | 1.0 |
| 4. | Glyceryl triisostearate | 5.0 |
| 5. | Stearic acid | 3.0 |
| 6. | Glyceryl monostearate | 1.5 |
| 7. | Pyrrolidonecarboxyl group-containing organopolysiloxane of Example 6 | 0.7 |
| 8. | Sorbitan sesquioleate | 0.5 |
| 9. | Polyoxyethylene sorbitan monooleate | 1.0 |
| 10. | Sodium hydroxide (1% aqueous solution) | 10.0 |
| 11. | 1,3-Butylene glycol | 5.0 |
| 12. | Preservative | appropriate amount |
| 13. | Perfume | appropriate amount |
| 14. | Purified water | 54.3 |

| | | |
|---|---|---|
| (Note 1) Acrylic silicone resin/decamethylcyclopentasiloxane: KP545 (made by Shin-Etsu Chemical Co., Ltd.) (Note 2) Stearyl-modified acrylic silicone: KP561 (made by Shin-Etsu Chemical Co., Ltd.) | | |

### (Preparation method)

A: Ingredients 1 to 9 were mixed and dissolved under heating conditions.
B: Ingredients 10 to 12 and 14 were mixed and heated.
C: B was added to A and emulsified, followed by cooling and further addition of ingredient 13 to obtain an O/W hand cream.

It was confirmed that the thus obtained hand cream was free of greasiness and was diffusively spread and run with excellent adherability and good fitness, and that the cream ensured a glossy finish and a very excellent makeup lasting performance and was very stable in temperature and aging changes.

### Example 33

Serum

| (Ingredients) | | (%) |
|---|---|---|
| 1. | Decamethylcyclopentasiloxane | 12.0 |
| 2. | Glyceryl triisooctanoate | 10.0 |
| 3. | Pyrrolidonecarboxyl group-containing organopolysiloxane of Example 7 | 2.0 |
| 4. | Pyrrolidonecarboxyl group-containing organopolysiloxane of Example 6 | 0.2 |
| 5. | Glycerine | 10.0 |
| 6. | Magnesium ascorbate phosphate | 3.0 |
| 7. | Sodium chloride | 2.0 |
| 8. | Preservative | appropriate amount |
| 9. | Perfume | appropriate amount |
| 10. | Purified water | 60.8 |

### (Preparation method)

A: Ingredients 1 to 4 were mixed under heating conditions.
B: Ingredients 5 to 8 and 10 were heated and dissolved uniformly.
C: Under agitation, B was gently added to A and emulsified, followed by cooling and further addition of ingredient 9 to obtain a serum.

It was confirmed that the thus obtained serum was fine in texture, was diffusively spread and run, and was free of greasiness and that the serum was felt moist and fresh, underwent no temperature and aging changes and was thus very excellent in stability.

### Example 34

Antiperspirant

| (Ingredients) | | (%) |
|---|---|---|
| 1. | Octamethylcyclopentasiloxane | 30.0 |
| 2. | Pyrrolidonecarboxyl group-containing organopolysiloxane of Example 7 | 1.0 |
| 3. | Polyoxyethylene sorbitan monooleate (20 E.O.) | 0.5 |
| 4. | Glycine salt of aluminium zirconium tetrachloride hydrate | 20.0 |
| 5. | Water | 48.5 |

### (Preparation method)

A: Ingredients 1, 2 were mixed.
B: Ingredient 4 was dissolved in ingredient 5, to which ingredient 3 was added.
C: Under agitation, B was gently added to A and emulsified to obtain an antiperspirant.

It was confirmed that the thus obtained antiperspirant was diffusively spread and was free of greasiness and oiliness and that the antiperspirant did not become too white and was felt as fresh in use and underwent no temperature and aging changes and was thus excellent in stability.

### Example 35

Cleansing cream

| (Ingredients) | | (%) |
|---|---|---|
| 1. | Dimethylpolysiloxane (6 mm²/s (25°C)) | 5.0 |
| 2. | Methylphenylpolysiloxane | 5.0 |
| 3. | Liquid paraffin | 8.0 |
| 4. | Jojoba oil | 2.0 |
| 5. | Pyrrolidonecarboxyl group-containing organopolysiloxane of Example 6 | 2.5 |
| 6. | Pyrrolidonecarboxyl group-containing organopolysiloxane of Example 7 | 0.5 |
| 7. | Dextrin fatty acid ester | 0.8 |
| 8. | Aluminum monostearate | 0.2 |
| 9. | Aluminum chloride | 1.0 |
| 10. | Glycerine | 10.0 |
| 11. | Preservative | appropriate amount |
| 12. | Perfume | appropriate amount |
| 13. | Purified water | 65.0 |

### (Preparation method)

A: Ingredients 1 to 8 were mixed under heating conditions.
B: Ingredients 9 to 11 and 13 were mixed under heating conditions.
C: Under agitation, B was gently added to A and emulsified, followed by cooling and further addition of ingredient 12 to obtain a cleansing cream.

It was confirmed that the thus obtained hand cream was fine in texture, was diffusively spread and run and was free of greasiness and that the cream was felt as moist, fresh and refreshed in use, showed a good cleansing effect and underwent no temperature or aging change, thus being excellent in stability.

### Example 36

Treatment gel

| (Ingredients) | | (%) |
|---|---|---|
| 1. | Ethanol | 20.0 |
| 2. | Pyrrolidonecarboxyl group-containing organopolysiloxane of Example 7 | 0.5 |
| 3. | Glyceryl triisooctanoate | 3.0 |
| 4. | Stearoxy-modified silicone (Note 1) | 2.0 |
| 5. | Silicone composite powder (Note 2) | 8.0 |
| 6. | Carboxyvinyl polymer (1% aqueous solution) | 20.0 |
| 7. | Triethanolamine | 0.2 |
| 8. | Preservative | appropriate amount |
| 9. | Perfume | appropriate amount |
| 10. | Purified water | 46.3 |

| | | |
|---|---|---|
| (Note 1) Stearoxy-modified silicone: KF-7002 (made by Shin-Etsu Chemical Co., Ltd.) (Note 2) Silicone composite powder: KSP-100 (made by Shin-Etsu Chemical Co., Ltd.) | | |

### (Preparation method)

A: Ingredients 1 to 5 were mixed and dispersed.
B: Ingredients 6 to 8 and 10 were added and mixed uniformly.
C: B was gently added to A, to which ingredient 9 was added and uniformly mixed.

It was confirmed that the resulting treatment gel was diffusively spread and run and was free of greasiness and oiliness and that the gel was felt as moist, fresh and refreshed in use, was well fit with the skin and underwent no temperature or aging change, thus being excellent in stability.

### Example 37

Rinse-type pack cosmetic

| (Ingredients) | | (%) |
|---|---|---|
| 1. | Dimethylsiloxane (6 mm²/s (25°C)) | 3.0 |
| 2. | Organopolysiloxane of Example 9 | 2.0 |
| 3. | Kaolin | 30.0 |
| 4. | Carboxyvinyl polymer | 0.4 |
| 5. | 1,3-Butylene glycol | 10.0 |
| 6. | Glycerine | 20.0 |
| 7. | Triethanolamine | 0.4 |
| 8. | Preservative | appropriate amount |
| 9. | Perfume | appropriate amount |
| 10. | Purified water | 34.2 |

### (Preparation method)

A: Ingredients 1, 2 and 8 were mixed together.
B: Ingredients 4 to 7 and 10 were added and mixed uniformly followed by addition of ingredient 3 and agitation.
C: B was added to A and emulsified, to which ingredient 9 was added thereby obtaining a pasty rinse-type pack cosmetic.

It was confirmed that the thus obtained rinse-type pack cosmetic was diffusively spread and run during application thereof and showed an excellent washing effect, and after rinsing, one felt moist and free of greasiness, leaving one's skin feeling silky smooth, thus ensuring a very excellent use feeling along with excellent stability.

### Example 38

Deodorant

| (Ingredients) | | (%) |
|---|---|---|
| 1. | Decamethylcyclopentasiloxane | 12.0 |
| 2. | Dimethylpolysiloxane (6 mm²/s (25°C)) | 4.0 |
| 3. | Pyrrolidonecarboxyl group-containing organopolysiloxane of Example 6 | 1.0 |
| 4. | Propylene glycol | 31.0 |
| 5. | Trichlosan | 0.1 |
| 6. | Glycerine | 15.0 |
| 7. | Sodium chloride | 0.1 |
| 8. | Preservative | appropriate amount |
| 9. | Perfume | appropriate amount |
| 10. | Purified water | 36.8 |

### (Preparation method)

A: Ingredients 1 to 3 were mixed.
B: Ingredient 5 was dissolved in ingredient 4, with which ingredients 6 to 8 and 10 were mixed.
C: While vigorously agitating A, B was added and emulsified, to which ingredient 9 was added.
D: 65 parts of C and 35 parts of a propellant (made of a mixture of n-butane, isobutene and propane) were charged into an aerosol can to obtain a deodorant.

It was confirmed that the thus obtained deodorant did not feel unpleasant when used in high concentration, was free of greasiness and was felt dry and that the deodorant ensured continued effect and thus had very excellent usability.

### Example 39

O/W/O milky lotion

| (Ingredients) | | (%) |
|---|---|---|
| 1. | Crosslinking polyether-modified silicone (Note 1) | 3.0 |
| 2. | Pyrrolidonecarboxyl group-containing organopolysiloxane of Example 7 | 1.0 |
| 3. | Glyceryl triisooctanoate | 14.0 |
| 4. | Crosslinking alkyl-modified silicone compound (Note 2) | 5.0 |
| 5. | Saccharose monostearate | 3.0 |
| 6. | Glycerine | 5.0 |
| 7. | 1,3-Butylene glycol | 5.0 |
| 8. | Preservative | appropriate amount |
| 9. | Purified water | 60.0 |
| 10. | Macadamia nut oil | 2.0 |
| 11. | Cetyl alcohol | 2.0 |
| 12. | Perfume | appropriate amount |

| | | |
|---|---|---|
| (Note 1) Crosslinking polyether-modified silicone: KSG-21 (made by Shin-Etsu Chemical Co., Ltd.) (Note 2) Crosslinking alkyl-modified silicone compound: KSG-43 (made by Shin-Etsu Chemical Co., Ltd.) | | |

### (Preparation method)

A: Ingredients 1 to 4 were mixed uniformly.
B: Ingredients 5 to 9 were mixed under heating conditions to provide a uniform mixture.
C: Ingredients 10 to 12 were mixed under heating conditions.
D: While agitating B, C was added thereto, emulsified and cooled.
E: While agitating A, D was added thereto and emulsified.

The thus obtained milky lotion was diffusively spread, was felt fresh, was free of greasiness and oiliness and was transparent, and that the lotion had a good makeup lasting performance, underwent no temperature or aging change and was very stable with respect to usability and stability.

### Example 40

O/W/O liquid foundation

| (Ingredients) | | (%) |
|---|---|---|
| 1. | Crosslinking polyether-modified silicone (Note 1) | 4.0 |
| 2. | Pyrrolidonecarboxyl group-containing organopolysiloxane of Example 6 | 1.0 |
| 3. | Propylene glycol decanoate | 5.0 |
| 4. | Isopropyl myristate | 5.0 |
| 5. | Pigment | 10.0 |
| 6. | Egg yolk-derived hydrogenated phospholipid | 1.0 |
| 7. | Glycerine | 2.0 |
| 8. | 1,3-Butylene glycol | 10.0 |
| 9. | Preservative | appropriate amount |
| 10. | Purified water | 52.0 |
| 11. | Squalane | 5.0 |
| 12. | Cetyl alcohol | 5.0 |
| 13. | Perfume | appropriate amount |

| | | |
|---|---|---|
| (Note 1) Crosslinking polyether-modified silicone: KSG-21 (made by Shin-Etsu Chemical Co., Ltd.) | | |

### (Preparation method)

A: Ingredients 1 to 4 were mixed uniformly.
B: Ingredients 5 to 10 were mixed under heating conditions to provide a uniform mixture.
C: Ingredients 11 to 13 were mixed under heating conditions.
D: While agitating B, C was added thereto, emulsified and cooled.
E: While agitating A, D was added thereto and emulsified.

The thus obtained liquid foundation was diffusively spread, was felt fresh, was free of greasiness and oiliness and was transparent, and that the liquid foundation had a good makeup lasting performance, underwent no temperature or aging change and was very stable with respect to usability and stability.

In respect of numerical ranges disclosed in the present description it will of course be understood that in the normal way the technical criterion for the upper limit is different from the technical criterion for the lower limit, i.e. the upper and lower limits are intrinsically distinct proposals.

## Claims

1. An organopolysiloxane compound having a weight average molecular weight of 300 to 200,000 determined by gel permeation chromatography using polystyrene stardards and having per molecule at least one organic group represented by the following formula (1) and at least one (R¹SiO_{3/2}) unit, wherein R¹ represents a hydrogen atom or an organic group selected from an alkyl group, a cycloalkyl group, a fluorine-substituted alkyl group, an aryl group, an aralkyl group, an organooxy group and a group represented by the following formula (1), and at least one R¹ is a group represented by the formula (1): 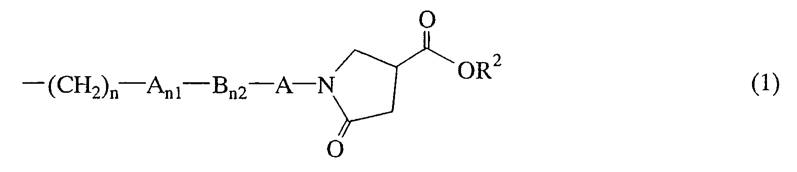 wherein R² represents a hydrogen atom, an alkyl group having 1 to 6 carbon atoms, or an alkali metal, A independently represents a linear or branched alkylene group having 1 to 12 carbon atoms, B represents -NR³-, sulfur or oxygen atom, in which R³ represents a hydrogen atom or an alkyl group having 1 to 6 carbon atoms, n is 0 or 2, n1 is 0 or 1, and n2 is 0 or 1 provided that (i) when n is 0 and n2 is 1, n1 is 1, (ii) when n is 2 and n2 is 1, n1 is 0 or 1, and (iii) when n is 2 and n2 is 0, n1 is 0.

2. An organopolysiloxane compound as defined in claim 1, wherein said organopolysiloxane compound is represented by the following formula (2):
I(R⁴₃SiO_{½})ₐ(R⁴₂SiO)_{b}(RiSO_{3/2})_{c}(SiO₂)_{d} (2)
wherein R¹ has the same meaning as defined above, R⁴ independently represents a hydrogen atom or an organic group selected from an alkyl group, a cycloalkyl group, a fluorine-substituted alkyl group, an aryl group, an aralkyl group, an organooxy group and a group represented by the formula (1), a is 3 to 50, b is 0 to 2,000, c is 1 to 30 and d is 0 to 30 provided that c+d is 1 or over.

3. An organopolysiloxane compound as defined in claim 2 in which in formula (2) c+d is 10 or below.

4. An organopolysiloxane compound as defined in claim 3 in which in formula (2) c is 1 to 5 and d is 0 to 3.

5. An organopolysiloxane compound as defined in any one of the preceding claims in which said weight average molecular weight is from 1,000 to 10,000.

6. An organopolysiloxane compound as defined in any one of the preceding claims in which the group of formula (1) is a group of one of formulae (7) to (9):

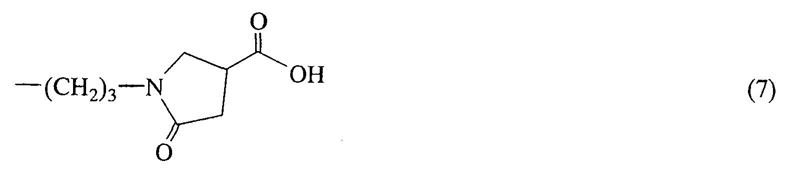

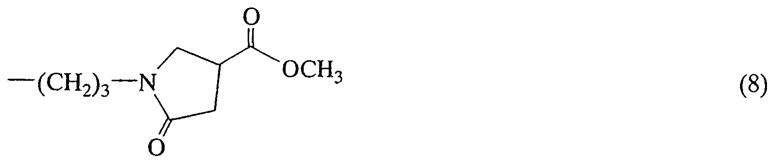

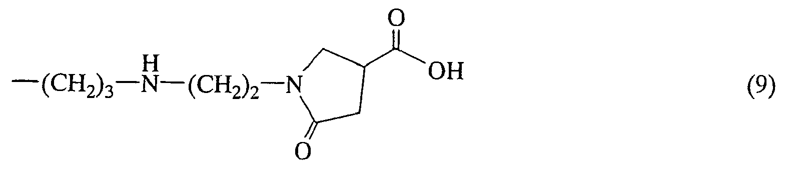

7. An amidoamine compound obtained by the reaction between the group of the formula (1) of an organopolysiloxane compound defined in any one of claims 1 to 6 and an amine compound represented by the following formula (3):
HNR⁵(CH₂)ₙ₃NR⁶R⁷ (3)
wherein R⁵ represents a hydrogen atom, an alkyl group, a hydroxyalkyl group, an alkenyl group, a cycloalkyl group or a polyoxyalkylene group, R⁶ and R⁷ may be the same or different and represent an alkyl group, a hydroxyalkyl group, a cycloalkyl group, a carboxyalkyl group or a polyoxyalkylene group, or R⁶ and R⁷ may form an N-heterocyclic ring along with the nitrogen atoms thereof, and n3 is an integer of 0 or over.

8. A cosmetic preparation comprising an organopolysiloxane compound according to any one of claims 1 to 6.

9. A cosmetic preparation as defined in claim 8, wherein said cosmetic preparation is a skin cosmetic.

10. A cosmetic preparation as defined in claim 8, wherein said cosmetic preparation is a hair cosmetic.

11. A cosmetic preparation according to any one of claims 8 to 10, wherein said cosmetic preparation comprises a powder treated with said organopolysiloxane compound.

12. A cosmetic preparation according to claim 11 in which said powder is titanium dioxide, zinc oxide, mica, sericite, talc or kaolin.

## Patentansprüche

1. Organopolysiloxanverbindung mit einem mittels Gelpermeationschromatographie unter Verwendung von Polystyrolstandards bestimmten gewichtsmittleren Molekulargewicht von 300 bis 200.000 und pro Molekül zumindest einer organischen Gruppe der folgenden Formel (1) sowie zumindest einer (R¹SiO_{3/2})-Einheit, worin R¹ für ein Wasserstoffatom oder eine organische Gruppe steht, die aus einer Alkylgruppe, einer Cycloalkylgruppe, einer fluorsubstituierten Alkylgruppe, einer Arylgruppe, einer Aralkylgruppe, einer Organooxygruppe und einer Gruppe der folgenden Formel (1) ausgewählt ist und zumindest ein R¹ eine Gruppe der Formel (1) ist: 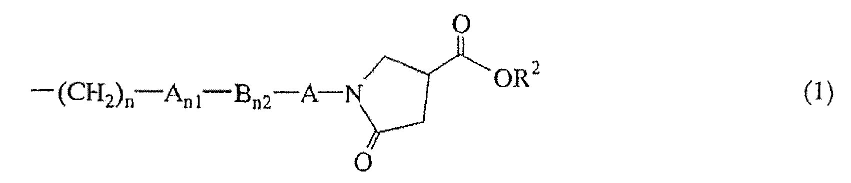 worin R² für ein Wasserstoffatom, eine Alkylgruppe mit 1 bis 6 Kohlenstoffatomen oder ein Alkalimetall steht, die A unabhängig voneinander für eine unverzweigte oder verzweigte Alkylengruppe mit 1 bis 12 Kohlenstoffatomen stehen, B für -NR³-, ein Schwefel- oder ein Sauerstoffatom steht, worin R³ für ein Wasserstoffatom oder eine Alkylgruppe mit 1 bis 6 Kohlenstoffatomen steht, n = 0 oder 2 ist, n1 = 0 oder 1 ist und n2 = 0 oder 1 ist, mit der Maßgabe, dass, (i) wenn n = 0 ist und n2 = 1 ist, n1 = 1 ist, (ii) wenn n = 2 ist und n2 = 1 ist, n1 = 0 oder 1 ist und (iii) wenn n = 2 ist und n2 = 0 ist, n1 = 0 ist.

2. Organopolysiloxanverbindung nach Anspruch 1, worin die Organopolysiloxanverbindung durch die folgenden Formel (2) dargestellt ist:
(R⁴₃SiO_{1/2})ₐ (R⁴₂SiO)_{b}(R¹SiO_{3/2})_{c}(SiO₂)_{d} (2)
worin R¹ wie oben definiert ist, die R⁴ unabhängig voneinander für Wasserstoffatome oder organische Gruppen stehen, die aus Alkylgruppen, Cycloalkylgruppen, fluorsubstituierten Alkylgruppen, Arylgruppen, Aralkylgruppen, Organooxygruppen und Gruppen der Formel (1) ausgewählt sind, a = 3 bis 50 ist, b = 0 bis 2.000 ist, c = 1 bis 30 ist und d = 0 bis 30 ist, mit der Maßgabe, dass c+d = 1 oder mehr ist.

3. Organopolysiloxanverbindung nach Anspruch 2, worin in Formel (2) c+d = 10 oder weniger ist.

4. Organopolysiloxanverbindung nach Anspruch 3, worin in Formel (2) c = 1 bis 5 ist und d = 0 bis 3 ist.

5. Organopolysiloxanverbindung nach einem der vorangegangenen Ansprüche, worin das gewichtsmittlere Molekulargewicht 1.000 bis 10.000 beträgt.

6. Organopolysiloxanverbindung nach einem der vorangegangenen Ansprüche, worin die Gruppe der Formel (1) eine Gruppe einer der folgenden Formeln (7) bis (9) ist:

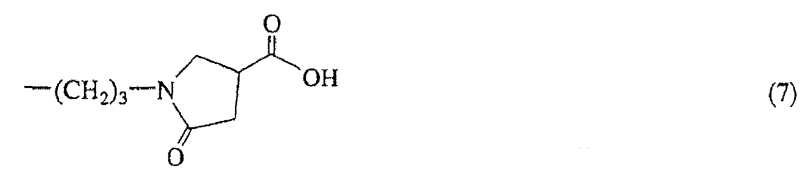

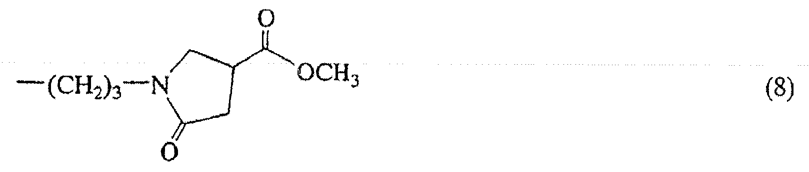

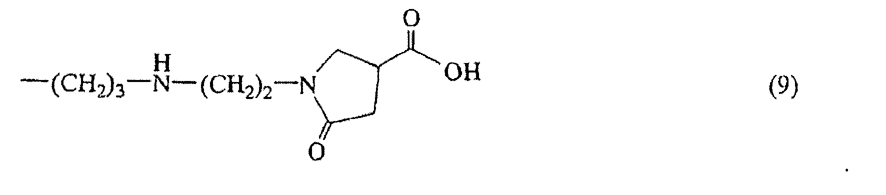

7. Amidoaminverbindung, erhalten durch Umsetzung einer Gruppe der Formel (1) einer Organopolysiloxanverbindung nach einem der Ansprüche 1 bis 6 mit einer Aminverbindung der folgenden Formel (3):
HNR⁵(CH₂)ₙ₃NR⁶R⁷ (3)
worin R⁵ für ein Wasserstoffatom, eine Alkylgruppe, eine Hydroxyalkylgruppe, eine Alkenylgruppe, eine Cycloalkylgruppe oder eine Polyoxyalkylengruppe steht, R⁶ und R⁷ gleich oder unterschiedlich sein können und für eine Alkylgruppe, eine Hydroxyalkylgruppe, eine Cycloalkylgruppe, eine Carboxyalkylgruppe oder eine Polyoxyalkylengruppe stehen oder R⁶ und R⁷ zusammen mit ihren Stickstoffatomen einen N-heterozyklischen Ring bilden, und n3 eine ganze Zahl von 0 oder mehr ist.

8. Kosmetisches Präparat, das eine Organopolysiloxanverbindung nach einem der Ansprüche 1 bis 6 umfasst.

9. Kosmetisches Präparat nach Anspruch 8, wobei das kosmetische Präparat ein Hautkosmetikum ist.

10. Kosmetisches Präparat nach Anspruch 8, wobei das kosmetische Präparat ein Haarkosmetikum ist.

11. Kosmetisches Präparat nach einem der Ansprüche 8 bis 10, wobei das kosmetische Präparat ein Pulver umfasst, das mit der Organopolysiloxanverbindung versetzt wurde.

12. Kosmetisches Präparat nach Anspruch 11, wobei das Pulver Titanoxid, Zinkoxid, Glimmer, Sericit, Talk oder Kaolin ist.

## Revendications

1. Composé organopolysiloxane ayant une masse moléculaire moyenne en masse de 300 à 200 000, déterminée par chromatographie par perméation sur gel utilisant des étalons de polystyrène, et ayant, par molécule, au moins un groupe organique représenté par la formule (1) suivante et au moins un motif (R¹SiO_{3/2}) où R¹ représente un atome d'hydrogène ou un groupe organique choisi parmi un groupe alkyle, un groupe cycloalkyle, un groupe fluoroalkyle, un groupe aryle, un groupe aralkyle, un groupe organoxy et un groupe représenté par la formule (1) suivante, et au moins un R¹ est un groupe représenté par la formule (1) : 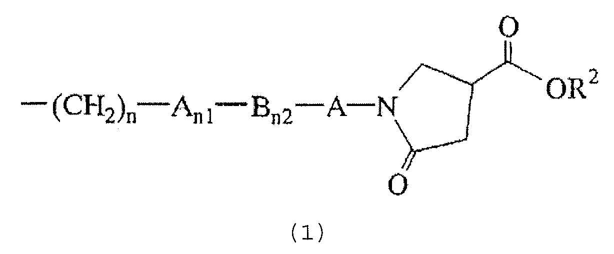 où R² représente un atome d'hydrogène, un groupe alkyle ayant 1 à 6 atomes de carbone, ou un métal alcalin, A représente indépendamment un groupe alkylène linéaire ou ramifié ayant 1 à 12 atomes de carbone, B représente -NR³- ou un atome de soufre ou d'oxygène, où R³ représente un atome d'hydrogène ou un groupe alkyle ayant 1 à 6 atomes de carbone, n vaut 0 ou 2, n1 vaut 0 ou 1, et n2 vaut 0 ou 1, à la condition que (i) lorsque n vaut 0 et n2 vaut 1, alors n1 vaille 1, (ii) lorsque n vaut 2 et n2 vaut 1, alors n1 vaille 0 ou 1, et (iii) quand n vaut 2 et n2 vaut 0, alors n1 vaille 0.

2. Composé organopolysiloxane selon la revendication 1, lequel composé organopolysiloxane est représenté par la formule (2) suivante :
(R⁴₃SiO_{1/2})ₐ (R⁴₂SiO)_{b} (R¹SiO_{3/2})_{c} (SiO₂)_{d} (2)
dans laquelle R¹ a la même signification que celle définie ci-dessus, R⁴ représente indépendamment un atome d'hydrogène ou un groupe organique choisi parmi un groupe alkyle, un groupe cycloalkyle, un groupe fluoroalkyle, un groupe aryle, un groupe aralkyle, un groupe organoxy et un groupe représenté par la formule (1), a vaut 3 à 50, b vaut 0 à 2 000, c vaut 1 à 30 et d vaut 0 à 30, à condition que c+d vaille 1 ou plus.

3. Composé organopolysiloxane selon la revendication 2, dans lequel, dans la formule (2), c+d vaut 10 ou moins.

4. Composé organopolysiloxane selon la revendication 3, dans lequel, dans la formule (2), c vaut 1 à 5 et d vaut 0 à 3.

5. Composé organopolysiloxane selon l'une quelconque des revendications précédentes, dans lequel ladite masse moléculaire moyenne en masse est de 1 000 à 10 000.

6. Composé organopolysiloxane selon l'une quelconque des revendications précédentes, dans lequel le groupe de formule (1) est un groupe de l'une des formules (7) à (9) :

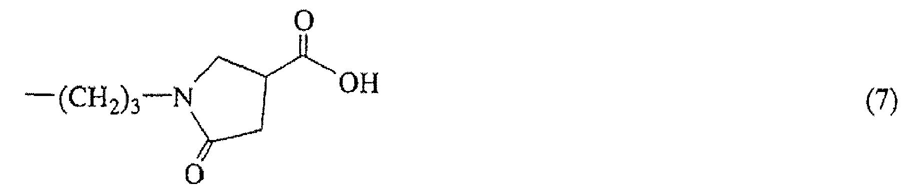

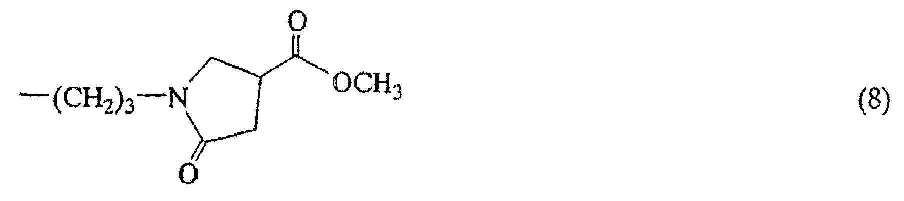

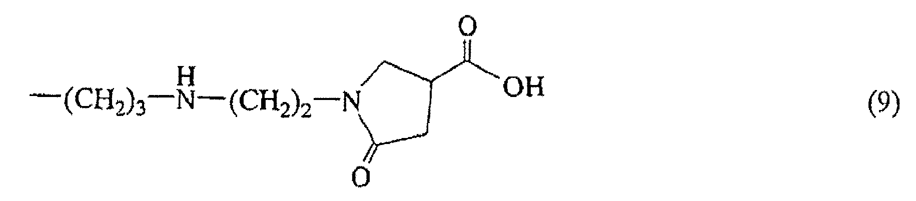

7. Composé amidoamine obtenu par la réaction entre le groupe de formule (1) d'un composé organopolysiloxane défini dans l'une quelconque des revendications 1 à 6 et un composé amine représenté par la formule (3) suivante :
HNR⁵(CH₂)ₙ₃NR⁶R⁷ (3)
dans laquelle R⁵ représente un atome d'hydrogène, un groupe alkyle, un groupe hydroxyalkyle, un groupe alcényle, un groupe cycloalkyle ou un groupe polyoxyalkylène, R⁶ et R⁷ peuvent être identiques ou différents et représentent un groupe alkyle, un groupe hydroxyalkyle, un groupe cycloalkyle, un groupe carboxyalkyle ou un groupe polyoxyalkylène, ou bien R⁶ et R⁷ peuvent former un cycle N-hétérocyclique conjointement avec leurs atomes d'azote, et n3 est un entier valant 0 ou plus.

8. Préparation cosmétique comprenant un composé organopolysiloxane selon l'une quelconque des revendications 1 à 6.

9. Préparation cosmétique selon la revendication 8, laquelle préparation cosmétique est un cosmétique pour la peau.

10. Préparation cosmétique selon la revendication 8, laquelle préparation cosmétique est un cosmétique pour les cheveux.

11. Préparation cosmétique selon l'une quelconque des revendications 8 à 10, laquelle préparation cosmétique comprend une poudre traitée avec ledit composé organopolysiloxane.

12. Préparation cosmétique selon la revendication 11, dans laquelle ladite poudre est le dioxyde de titane, l'oxyde de zinc, le mica, la séricite, le talc ou le kaolin.
